(19) Europäisches Patentamt
European Patent Office
Office européen des brevets

(11) Veröffentlichungsnummer : **0 131 181**
**B1**

(12) # EUROPÄISCHE PATENTSCHRIFT

(45) Veröffentlichungstag der Patentschrift : **07.03.90**

(21) Anmeldenummer : **84106995.8**

(22) Anmeldetag : **19.06.84**

(51) Int. Cl.$^5$ : **C 07 H 15/24**, A 61 K 31/70, C 12 P 19/56 // (C12P19/56, C12R1:465)

(54) **Anthracyclin-Derivate, ein mikrobiologisches Verfahren zu ihrer Herstellung und ihre Verwendung als Cytostatika.**

(30) Priorität : **25.06.83 DE 3323025**

(43) Veröffentlichungstag der Anmeldung : **16.01.85 Patentblatt 85/03**

(45) Bekanntmachung des Hinweises auf die Patenterteilung : **07.03.90 Patentblatt 90/10**

(84) Benannte Vertragsstaaten : **AT BE CH DE FR GB IT LI LU NL SE**

(56) Entgegenhaltungen :
**EP–A– 0 078 447**
**EP–A– 0 110 155**
**FR–A– 2 362 157**
**US–A– 4 316 011**
**JOURNAL OF MEDIC. CHEMISTRY, Band 23, 1980, American Chemical Society, (US) Seiten 1242-1244 H. LIN et al.: "8-Hydroxyanthracyclinones from epsilon-Rhodemycinone"**
**THE JOURNAL OF ANTIBIOTICS, Band XXXIII, Nr. 11, November 1980, Tokyo (JP) T. OKI et al.: "Biosyntheseses of anthracycline antibiotics by streptomyces galilaeus I. Glycosidation of various anthracyclinones by an aclacinomycinnegative mutant and biosynthesis of aclacinomycins from aklavinone" Seiten 1331-1340**

(73) Patentinhaber : **HOECHST AKTIENGESELLSCHAFT**
**Postfach 80 03 20**
**D-6230 Frankfurt am Main 80 (DE)**

(72) Erfinder : **Aretz, Werner, Dr.**
**Am Krummorgen 2**
**D-6233 Kelkheim (Taunus) (DE)**
Erfinder : **Berscheid, Hans Gerd, Dr.**
**Rheinlandstrasse 21**
**D-6231 Schwalbach (DE)**
Erfinder : **Huber, Gerhard, Dr.**
**In den Bleichwiesen 2**
**D-6233 Kelkheim (Taunus) (DE)**
Erfinder : **Fehlhaber, Hans-Wolfram, Dr.**
**Thomas-Mann-Strasse 5a**
**D-6270 Idstein/Taunus (DE)**
Erfinder : **Kraemer, Hans Peter, Dr.**
**Birkenweg 16**
**D-3550 Marburg (DE)**
Erfinder : **Sedlacek, Hans-Harald, Dr.**
**Am Sonnenhang 3**
**D-3550 Marburg (DE)**
Erfinder : **Ganguli, Bimal Naresh, Dr.**
**"Saurayuth", Central Avenue 173**
**Chembure, Bombay, 400 071 (IN)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 131 181 B1

Jouve, 18, rue St-Denis, 75001 Paris, France

TETRAHEDRON LETTERS, Nr. 11, Pergamon Press, GB H. BROCKMANN et al.: "Zur Kenntnis der beta-Rhodomycine", Seiten 831-875
DIE PHARMAZIE, Band 27, Heft 10, Oktober 1972, Seiten 782-789, Berlin, DE; E. BIEDERMANN et al.: "Untersuchungen zur Isolierung und Kostitutionsaufklärung der Rhodomycin-Antibiotica des Streptomyces-Stammes JA 8467"
THE JOURNAL OF ANTIBIOTICS, Band 36, Nr. 8, August 1983, Seiten 1080-1083, Tokyo, JP; T. UCHIDA et al.: "New antitumor antibiotics, ditrisarubicins A, B and C"

Die Akte enthält technische Angaben, die nach dem Eingang der Anmeldung eingereicht wurden und die nicht in dieser Patentschrift enthalten sind.

Erfinder : Sood, Ratan Sagar, Dr.
c/o Dr. Nutan Sood, Chembure Children's Home
Mankhurd Bombay, 400 088 (IN)
Erfinder : Gandhi, Julia, Dr.
"Charisma", K. Hill Road 128, Somt+Somnath Lane
Bandra Bombay, 400 050 (IN)
Erfinder : Reddy, Gauknapalli Chandrasekhar, Dr.
Flat 104, Aarati Apartments, Sarojini Naidu Road
Mulund (West), Bombay 400 080 (IN)

## Beschreibung

Die vorliegende Erfindung betrifft neue Verbindungen mit antibakterieller und cytostatischer Wirkung sowie ihre mikrobiologische Herstellung.

Die Verbindungen, die sich insbesondere durch antibakterielle Wirkung gegen grampositive Bakterien und durch eine sehr gute Wirksamkeit gegen verschiedene Tumorarten auszeichnen, besitzen die Struktur

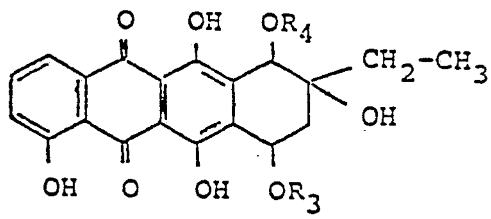

worin, falls $R_4$ für die Zuckerkombination Roa-Rod-Rod steht, $R_3$ die Bedeutung Roa-Rod-Rod, Roa-dF-Cin A oder Roa-Rod-Acu hat, oder falls $R_4$ für die Zuckerkombination Roa-Rod-Acu steht, $R_3$ die Bedeutung Roa-Rod-Acu oder Roa-dF-Cin A hat, oder, falls $R_4$ für die Zuckerkombination Roa-dF-Rod steht, $R_3$ die Bedeutung Roa-Rod-Rod oder Roa-dF-Rod hat, wobei

| | |
|---|---|
| Roa | Rhodosamin |
| dF | Desoxyfucose |
| Rod | Rhodinose |
| Acu | Aculose, und |
| Cin A | Cinerulose A |

bedeutet, wobei auch die physiologisch unbedenklichen Säureadditionssalze umfaßt sind.

Die vorliegende Erfindung betrifft ferner ein Verfahren zur Herstellung der genannten Verbindungen der Formel I durch Fermentation von Streptomyces Y-11472 (DSM 2658) mit nachfolgender Isolierung und Reinigung, sowie den Mikroorganismus-Stamm Y-11472 (DSM 2658).

Streptomyces Y-11472 wurde auf bekannte Art und Weise aus einer Bodenprobe unter Verwendung eines Nährmediums bei einem pH von 6,5 bis 8,5 mit oder ohne Zusatz von Antibiotika isoliert. Vorzugsweise wird Streptomyces Y-11472 aus einer Bodenprobe unter Verwendung eines Nährmediums bei einem pH von 6,5-7,0 isoliert. Als Antibiotika, die gegebenenfalls zum Nährmedium gegeben werden, nimmt man vorzugsweise « Ampicillin » oder « Amphotericin B ». Die Antibiotika verhindern Bakterien- und Pilzwachstum. Weiterhin können als Antibiotika « Streptomycin », « Penicillin » oder « Nystatin » eingesetzt werden.

Das Nährmedium besteht aux Kohlenstoff- und Stickstoffquellen sowie anorganischen Nährsalzen. Als Kohlenstoffquellen eignen sich Glucose oder Stärke. Als Stickstoffquellen kann man Pepton, Hefeextrakt, Fleischextrakt, Malzextrakt oder Casein verwenden. Zur Verfestigung kann man Agar verwenden. Als anorganische Nährsalze eignen sich beispielsweise Natrium-, Kalium, Magnesium-, Calcium-, Phosphor- oder Schwefelsalze.

Der so erhaltene Mikroorganismus Streptomyces Y-11472 wurde am 24.5.1983 unter der Eingangsnummer DSM 2658 bei der DSM (Deutsche Sammlung von Mikroorganismen) hinterlegt.

Der Mikroorganismus Streptomyces Y-11472 gehört zur Ordnung Actinomycetales, zur Familie der Streptomycetaceae und zur Gattung Streptomyces. Von verschiedenen in der Literatur beschriebenen Streptomyces-Arten ist bekannt, daß sie Anthracyclinverbindungen herstellen. Von diesen Arten werden bereits Daunomycin und Adriamycin in der Klinik am Menschen zur Krebsbekämpfung eingesetzt.

Rhodomycinone, iso-Rhodomycinon sowie Rhodomycin-verwandte Antibiotika sind beschrieben in Chem. Ber. 88, 1782-1818 (1955) ; Chem. Ber. 101, 1341-1348 (1968) ; J. Med. Chem. 20, 957-960 (1977) ; Pharmacie 27, 782-789 (1972) ; Zeit. Allg. Mikrobiol., 14, 551-558 (1974) ; Tetrahed. Lett. No. 38, 3699-3702 (1973) ; Folia Microbiol., 24, 293-295 (1979) ; and J. Antibiotics, 32, 420 (1979).

Aclacinomycin A ist beschrieben im US Patent Nr. 3 988 315 sowie von Oki et al. im J. Antibiotics 28, 830 (1975) und 32, 791-812 (1979).

Die Cinerubine A und B sind beschrieben im britischen Patent Nr. 846 130, im US Patent Nr. 3 864 480, in « Antimicrobial Agents and Chemotherapy », S. 68 (1970) von Keller-Schierlein et al., in Chemical Abstracts 54, 1466i (1960) und im J. Antibiotics 28, 830 (1975).

Weitere Anthracyclinantibiotika sind detailliert oder als Zusammenfassung im « Index of Antibiotics from Actinomycetes » Hauptherausgeber Hamao Umezawa, University Park Press, State College, Pennsylvania, USA (1967) wie folgt beschrieben worden :

| Antibiotika | Seitennummer |
|---|---|
| Aclacinomycine A und B | 101-102 |
| Adriamycin | 122 |
| Carminomycin I | 225 |
| Galirubine S-D | 405-408 |
| Rhodomycine X-Y | 879-880 |
| β-Rhodomycine | 881-885 |
| γ-Rhodomycine | 886-892 |
| Steffimycin | 945 |

In « Antibiotics », Vol. I Mechanisms of Action, Herausgeber David Gottlieb und Paul D. Shaw, Springer-Verlag New York, Inc., N.Y. (1967) ist auf den Seiten 190-210 eine Abhandlung von A. DiMacro mit dem Titel « Daunomycin and Related Antibiotics », enthalten.

Das « Information Bulletin », Nr. 10, International Center of Antibiotics, in Zusammenarbeit mit der WHO, Dezember 1972, Belgien, berichtet über Anthracycline und ihre Derivate.

Als nächster Stand der Technik kommen die folgenden Veröffentlichungen in Betracht :

EP-A-0 110 155 (Stand der Technik nach Art. 54(3) und (4) EPÜ) : Es werden Anthracyclinon-Derivate beschrieben, welche in 7-Stellung die Zuckerkombinationen Roa-dF=CinB und in 10-Stellung die Zuckerkombinationen Roa-dF-CinA, Roa-dF=CinB oder Roa-Rod-Acu tragen.

In Tetrahedron Letters, Nr. 11 (1975), S. 831-834 werden Anthracyclinon-Derivate beschrieben, welche in 7- und 10-Stellung Mono-, Di- oder Trisaccharid-Reste enthalten, z. B. die Kombination Roa-dF-Rod in 7- und die Kombination Roa-Rod-Rod in 10-Stellung.

EP-A-0 078 447 betrifft 10-Hydroxy-Anthracyclinon-Derivate mit den Zuckerkombinationen Roa-dF oder Roa-dF-Rod in 7-Stellung. US-A-4 316 011 betrifft Anthracyclinon-Derivate bzw. 1-Hydroxy-Anthracyclinon-Derivate, mit der Zuckerkombination Roa-dF-CinA in 7-Stellung und/oder einer der beiden Zuckerkombinationen Roa-dF-Rod und Roa-dF-CinA in 10-Stellung.

In Pharmazie 27, Heft 12 (1972), S. 782-789 schließlich werden in 7-Stellung unsubstituierte oder mit einer OH-Gruppe substituierte Anthracyclinon-Derivate mit der Zuckerkombination Roa-dF-Rod in 10-Stellung beschrieben.

Die Eigenschaften von Streptomyces Y-11472 werden in der Tabelle 2 dieser Beschreibung beschrieben.

Die vorliegende Erfindung betrifft auch ein Verfahren zur Herstellung von Verbindungen der Anthracyclinklasse der allgemeinen Formel I, durch Kultivieren von Streptomyces Y-11472 durch Fermentation bei einem pH-Wert von 6,5-8,5 und einer Temperatur von 24-40 °C unter aeroben Bedingungen in einem Kohlenstoff- und Stickstoffquellen sowie anorganische Nährsalze sowie Spurenelemente enthaltenden Nährmedium und Isolierung der Verbindungen aus der Kulturflüssigkeit und dem Mycel auf bekannte Art und Weise wie nachfolgend beschrieben.

Als Kohlenstoffquellen eignen sich Glucose, Stärke, Dextrin und Glycerin. Geeignete Stickstoffquellen sind Sojamehl, Hefeextrakt, Fleischextrakt, Malzextrakt, Maisquellwasser, Pepton oder Casein. Als anorganische Nährsalze eignen sich z. B. Natriumchlorid, Magnesiumsulfat oder Calciumkarbonat. Als Spurenelemente kann man Eisen, Magnesium, Kupfer, Zink und Kobalt verwenden.

Streptomyces Y-11472 kann bei Temperaturen von 24-40 °C bei einem pH von 6,5-8,5 kultiviert werden. Vorzugsweise erfolgt die Kultivierung des Streptomyces Y-11472 unter aeroben Bedingungen bei 30 °C und bei einem pH-Wert von 7,0. Nach 72 Stunden, wenn die höchste Ausbeute erreicht ist, bricht man die Fermentation ab. Vorzugsweise kann es sich bei der Fermentation um eine Submers-Fermentation handeln.

Der Fortschritt der Fermentation und die Bildung der Anthracyclinverbindungen kann anhand der antibakteriellen Wirksamkeit der Kulturflüssigkeit gegen S. aureus 209 P und Bac. subtilis sowie durch Extraktion der gesamten Kulturlösung (Mycel und Kulturfiltrat) mit einem organischen Lösungsmittel und Messung der Absorptionsintensität der roten Verbindung bei 494 nm verfolgt werden. Als organisches Lösungsmittel verwendet man vorzugsweise Äthylacetat.

Die Anthracyclinverbindungen im Kulturfiltrat und im Mycel werden gemäß dem Schema im Schaubild I der vorliegenden Erfindung isoliert.

Die Anthracyclinverbindungen im Mycel werden mit einem organischen Lösungsmittel, vorzugsweise mit wäßrigem Aceton, das auf einen pH-Wert von 3,5 eingestellt wurde, extrahiert. Nach Entfernung des Acetons stellt man den pH-Wert der wäßrigen Phase auf 7,5 ein und extrahiert dann mit Äthylacetat. Die Kulturflüssigkeit extrahiert man bei einem pH-Wert von 7,5 mit einem organischen Lösungsmittel wie Äthylacetat oder Chloroform, vorzugsweise mit Äthylacetat. Die Äthylacetatextrakte aus dem Mycel und dem Kulturfiltrat werden vereinigt oder separat aufgearbeitet, konzentriert und in einem organischen Lösungsmittel, wie Benzol oder Toluol, aufgenommen. Vorzugsweise verwendet man Toluol. Die Toluollösung extrahiert man dann mit einem Acetatpuffer von einem pH-Wert von 3,5. Auf dieser Stufe teilt man das Gemisch der Antracyclin-Derivate in zwei Fraktionen auf. Das in der Toluolphase verbleibende Gemisch bezeichnet man als Fraktion A, das in der wäßrigen Phase verbleibende Glykosidgemisch bezeichnet man als Fraktion B.

Die Fraktion B wird weiter gereinigt gemäß Schaubild II der vorliegenden Erfindung.

Wie im Schaubild II dargestellt, erhält man aus der Fraktion B die Cytorhodine A, B, F und das Gemisch N + O, P, V und W. Bei der Fraktion « X » handelt es sich um Mischungen aus anderen Anthracyclinverbindungen, die noch untersucht werden.

Schaubild I

Isolierung des antibiotischen Komplexes aus Streptomyces Y-11472

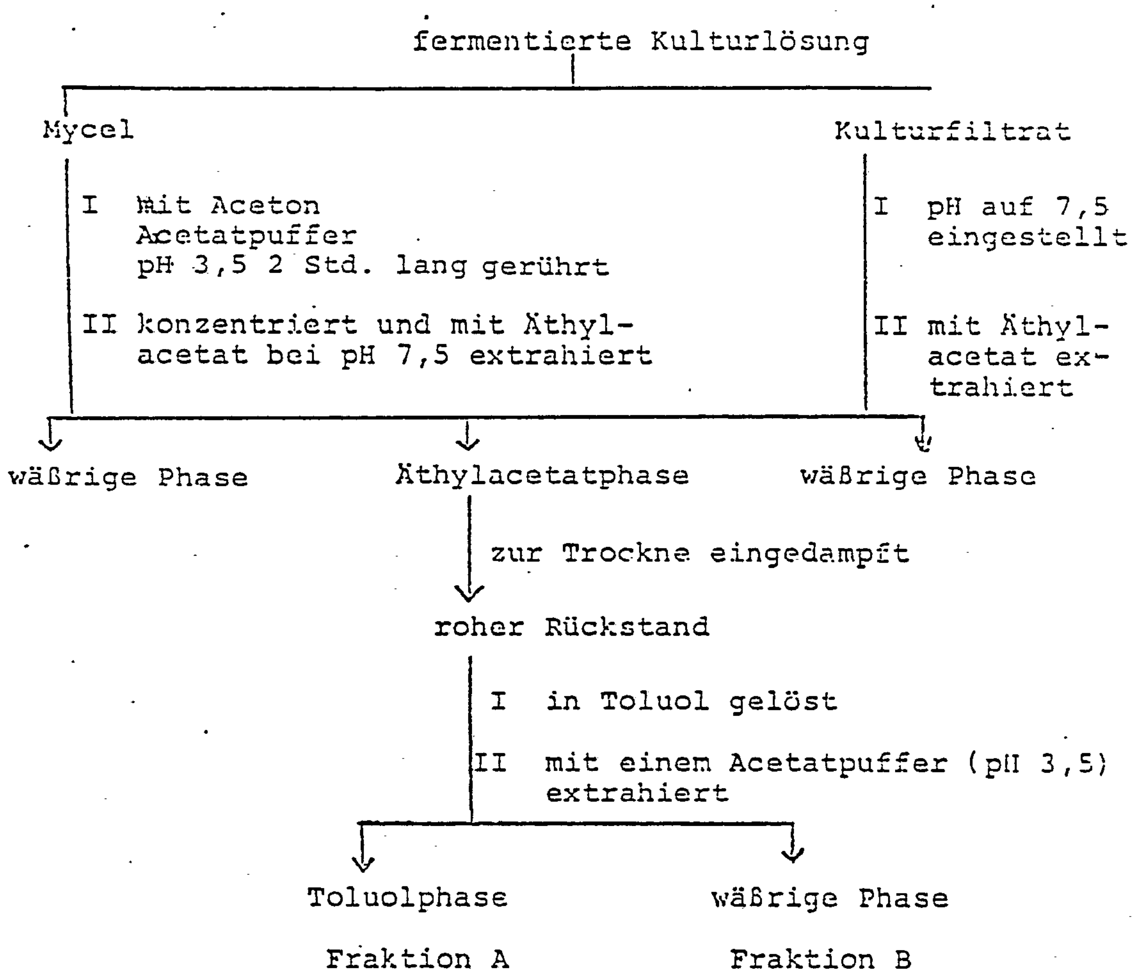


(Siehe Tabelle Seite 6 f.)

Schaubild II

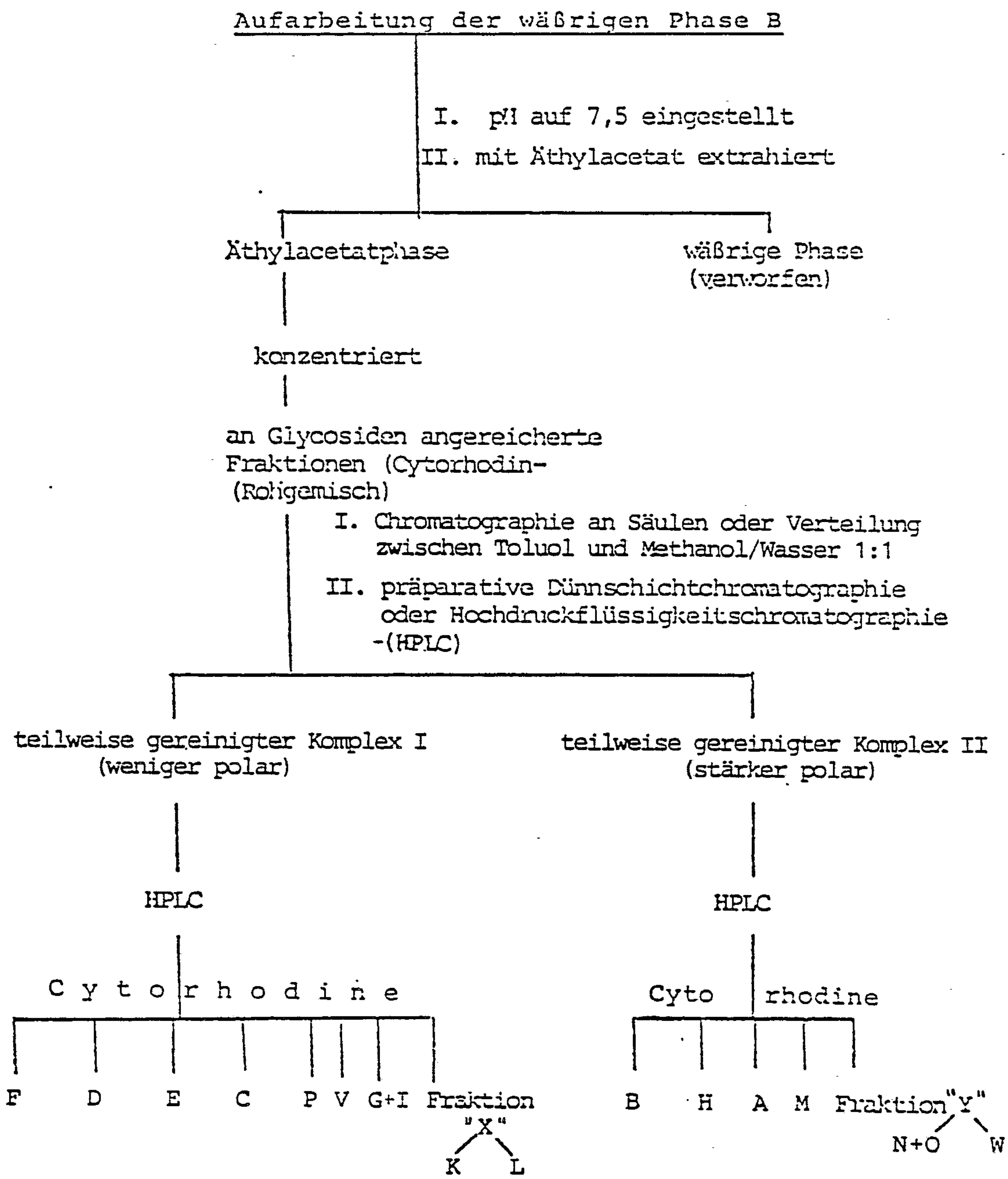


Die erfindungsgemäßen Verbindungen der Formel I

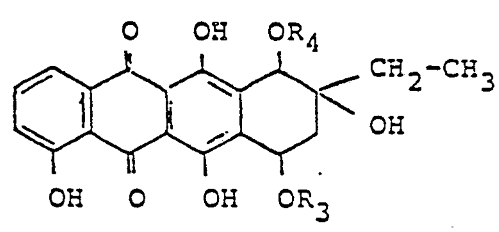

(I)

sind in der folgenden Tabelle aufgeführt, wobei $R_3$ und $R_4$ die im folgenden angegebenen Bedeutungen haben :

| Verbindung | | $R_3$ | $R_4$ |
|---|---|---|---|
| Cytorhodin | F | Roa-Rod-Acu (Formel V) | Roa-Rod-Acu (Formel V) |
| " | B | Roa-dF-Cin A (Formel VII) | Roa-Rod-Rod (Formel VIII) |
| " | A | Roa-Rod-Rod (Formel VIII) | Roa-Rod-Rod (Formel VIII) |
| " | P | Roa-dF-Cin A (Formel VII) | Roa- Rod-Acu (Formel V) |
| " | V | Roa-Rod-Acu (Formel V) | Roa-Rod-Rod (Formel VIII) |
| " | N | Roa-Rod-Rod (Formel VIII) | Roa-dF-Rod (Formel IX) |
| " | W | Roa-dF-Rod (Formel IX) | Roa-dF-Rod (Formel IX) |

Die erfindungsgemäßen Verbindungen fallen bei dem vorstehenden beschriebenen Verfahren zusammen mit anderen, bereits bekannten Cytorhodinen an, z. B. mit solchen der Formel I der folgenden Zusammensetzung

| Verbindung | | $R_3$ | $R_4$ |
|---|---|---|---|
| Cytorhodin | D | Roa-dF=Cin B (Formel VI) | Roa-Rod-Acu (Formel V) |
| " | C | Roa-dF=Cin B (Formel VI) | Roa-dF=Cin B (Formel VI) |
| " | G | Roa-dF=Cin B (Formel VI) | Roa-dF-Cin A (Formel VII) |
| " | I | Roa-dF-Cin A (Formel VII) | Roa-dF= Cin B (Formel VI) |
| " | O | Roa-dF-Rod (Formel IX) | Roa-Rod-Rod (Formel VIII) |

sowie mit solchen der Formel

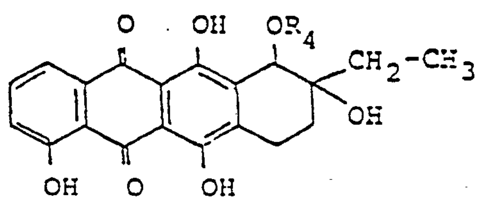

(II)

worin $R_4$ die folgenden Bedeutungen hat :

| Verbindung | | $R_4$ |
|---|---|---|
| Cytorhodin | E | Roa-Rod-Rod (Formel VIII) |
| " | K | Roa-dF-Rod (Formel IX) |
| " | L | Roa-Rod (Formel X) |
| " | M | Roa-dF (Formel XI) |

Die vorstehend genannten Zuckerkombinationen haben die folgenden Strukturformeln (V bis XI) :

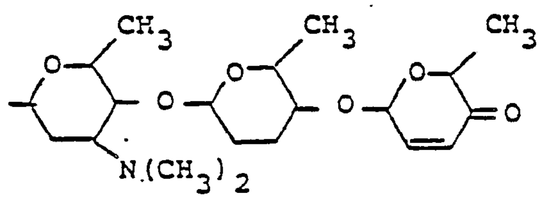

Roa-Rod-Acu

(V)

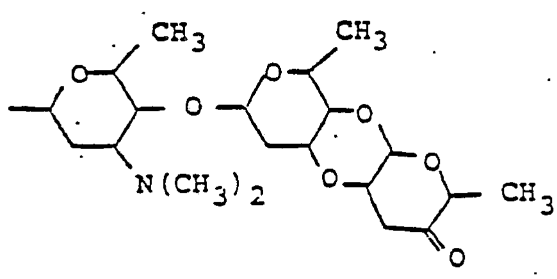

Roa-dF=Cin B

(VI)

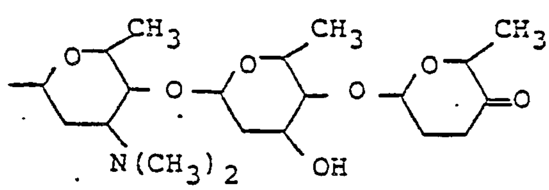

Roa-dF-Cin A

(VII)

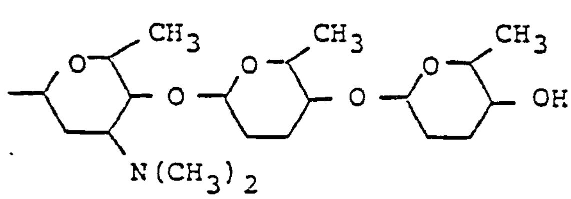

Roa-Rod-Rod

(VIII)

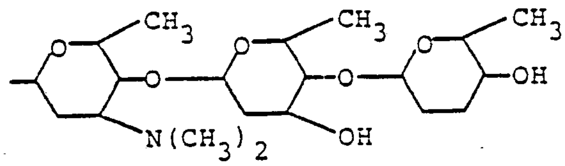

Roa-dF-Rod

(IX)

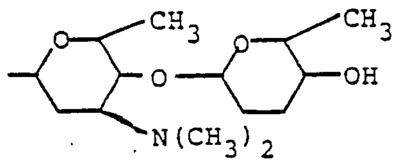

Roa-Rod

(X)

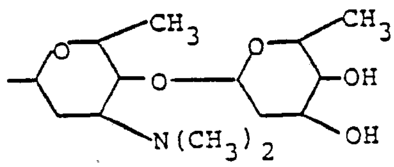

Roa-dF

(XI)

Die erfindungsgemäßen Anthracyclinverbindungen zeichnen sich durch eine starke Wirkung gegen grampositive Bakterien sowie eine ausgeprägte cytostatische Wirkung aus.

Die vorliegende Erfindung wird durch die nachfolgenden Beispiele näher erläutert :

Beispiel 1

Isolierung von Streptomyces Y-11472 aus dem Boden

a) Herstellung von Isolierungsnährmedien

Medium 1

| | |
|---|---|
| Glucose | 1.0 g |
| Glycerin | 1.0 g |
| L-Arginin | 0.3 g |
| $H_2HPO_4$ | 0.3 g |
| $MgSO_4 \cdot 7H_2O$ | 0.2 g |
| NaCl | 0.3 g |
| Hefeextrakt | 2.0 g |
| $Fe_2(SO_4)_3$ | 10 mg |
| $CuSO_4 \cdot 5H_2O$ | 1 mg |
| $ZnSO_4 \cdot 7H_2O$ | 1 mg |
| $MnSO_4 \cdot 7H_2O$ | 1 mg |
| Agar | 15 mg |
| destilliertes Wasser | 1 l |
| pH | 6.5 |

Medium 2

| | |
|---|---|
| Glucose | 2.0 g |
| L-Asparagin | 1.0 g |
| $K_2HPO_4$ | 0.5 g |
| $MgSO_4 \cdot 7H_2O$ | 0.5 g |
| Bodenextrakt | 200 ml |
| Agar | 15 g |
| destilliertes Wasser | 800 ml |
| pH | 8.0 |

Medium 3

| | |
|---|---|
| Stärke | 10.0 g |
| Casein | 0.3 g |
| $KNO_3$ | 2.0 g |
| NaCl | 2.0 g |
| $K_2HPO_4$ | 2.0 g |
| $MgSO_4$ | 0.05 g |
| $CaCO_3$ | 0.02 g |
| $FeSO_4$ | 0.01 g |
| Agar | 15 g |
| destilliertes Wasser | 1 l |
| pH | 7.2-7.5 |

Die Medien wurden 1/2 Stunde bei 121 °C sterilisiert.

b) Herstellung einer Bodenproben-Suspension

1 Gramm Bodenprobe wurde an der Luft getrocknet, in einer Reibschale zermahlen und 1 Stunde auf 120 °C erhitzt. Die so behandelte Bodenprobe wurde in destilliertem Wasser suspendiert und gründlich geschüttelt. Dann ließ man die Erde absitzen und benutzte die überstehende Flüssigkeit als Inokulum für die obigen Isoliermedien.

c) Beimpfung der Isolierungsmedien

1 ml Bodenprobe-Suspension wurde auf Petrischalen mit je 50 ml der obigen Isolierungsmedien beimpft.

d) Isolierung von Streptomyces Y-11472

Die beimpften Petrischalen wurden 2 Wochen bei 37 °C inkubiert und Streptomyces Y-11472 wurde auf bekannte Weise aus den wachsenden Mikroorganismen isoliert.

Beispiel 2

Kultivierung von Streptomyces Y-11472 zur fermentativen Produktion des antibiotischen Komplexes

Streptomyces Y-11472 wurde auf Hefe-Malz-Agar der folgenden Zusammensetzung gegeben :

| | |
|---|---|
| Malzextrakt | 10,0 g |
| Hefeextrakt | 4,0 g |
| Glucose | 4,0 g |
| Agar | 15,0 g |
| destilliertes Wasser | 1 l |
| pH | 7,0 |

Das Medium wurde auf Teströhrchen verteilt und 30 Min. bei 121 °C sterilisiert, dann wurden die Röhrchen in Schräglage zur Herstellung von Schrägkulturen abgekühlt, mit der Kultur beimpft und 10-15 Tage bei 28 °C inkubiert. Danach konnte man ein gutes Wachstum und eine gute Sporenbildung feststellen. Eine Sporensuspension in destilliertem Wasser aus einem Schrägröhrchen wurde als Inokulum für 5 Erlenmeyerkolben mit je 100 ml Impfkulturmedium oder für eine 5 l Saugflasche mit 1 l desselben Stammkulturmediums, verwendet.

Zusammensetzung des Impfkulturmediums

| | |
|---|---|
| Glucose | 15,0 g |
| Sojamehl | 15,0 g |
| Maisquellwasser | 5,0 g |
| $CaCO_3$ | 2,0 g |
| NaCl | 5,0 g |
| destilliertes Wasser | 1 l |
| pH | 7.0 |

Je 100 ml des obigen Mediums wurden auf 500 ml Erlenmeyerkolben verteilt oder 1 l des Mediums wurde in eine 5 l Saugflasche gegeben und das Medium wurde 30 Minuten bei 121 °C sterilisiert. Die

Kolben bzw. Flaschen wurden gekühlt, mit der Sporensuspension beimpft und bei 240 Upm 72 Std. bei 30 °C in einer Rotationsschüttelmaschine mit einer Amplitude von 3,75 cm geschüttelt. Die gewachsene Kultur wurde als Inokulum für einen 15 l Glasfermenter mit 10 l eines 5 Vol.-%igen Stammkulturmediums zur Herstellung einer Impfkultur für die 2. Stufe eingesetzt. Die Fermentation erfolgte bei 26 °C (± 1 °C) unter Rühren bei 160-180 Upm mit einer Belüftungsrate von 6-7 l/Min. Die dabei erhaltene gut angewachsene Impfkultur wurde als Inokulum für das Produktionsmedium verwendet.

Zusammensetzung des Produktionsmediums

| | |
|---|---|
| Glucose | 20,0 g |
| Malzextrakt | 10,0 g |
| Hefeextrakt | 4,0 g |
| destilliertes Wasser | 1 l |
| pH | 6,8 |

Die Ansätze der Fermenter wurden mit 0,025 % Desmophen® als Antischaum-Mittel versetzt.

260 l des obigen Mediums wurden in einem Fermenter von 390 l gegeben. Das Medium wurde durch indirekten sowie durch direkten Dampf 28 Min. bei 121 °C sterilisiert. Der Fermenter wurde abgekühlt und mit der Zweitstufenimpfkultur (5 Vol.-%) beimpft. Die Fermentation erfolgte 68-72 Std. bei 26 °C (± 1 °C) unter Rühren bei 100-200 Upm bei einer Belüftungsrate von 1 : 0,6 VVm (9-10 $m^3$/h). Bei Beendigung der Fermentation nach 68-72 Std. betrug die Konzentration des Antibiotikums 100-150 µg per ml und der pH der Kulturflüssigkeit 5,5-6,0. Der Restzuckergehalt in der Kulturlösung betrug 0,03-0,5 % und das Naßgewicht des Mycels betrug 3-4 g/Vol %.

## Beispiel 3

Anzucht von Streptomyces Y-11472 zur fermentativen Herstellung des antibiotischen Komplexes

a) (1) Zusammensetzung des Volkulturmediums

| | |
|---|---|
| Gramimpfpulver | 10,0 g |
| Dextrose | 10,0 g |
| NaCl | 5,0 g |
| $CaCO_3$ | 3,0 g |
| destilliertes Wasser | 1 l |
| pH | 7.0 |
| 72 Std. bei 30 °C. | |

(2) Zusammensetzung des Produktionsmediums

| | |
|---|---|
| Stärke | 10,0 g |
| Glucose | 10,0 g |
| Malzextrakt | 7,5 g |
| Pepton | 7,5 g |
| NaCl | 3,0 g |
| $MgSO_4$ | 1,0 g |
| $KH_2PO_4$ | 2,0 g |
| $CuSO_4 \cdot 5H_2O$ | 0,007 |
| $FeSO_4 \cdot 7H_2O$ | 0,001 |
| $MnSO_4 \cdot 4H_2O$ | 0,008 |
| $ZnSO_4 \cdot 7H_2O$ | 0,002 |
| destilliertes Wasser | 1 l |
| pH | 7,0 |
| Fermentationsdauer : 88-96 Std. | |

b) (1) Zusammensetzung des Vorkulturmediums

| | |
|---|---|
| Malzextrakt | 10,0 g |
| Hefeextrakt | 4,0 g |
| Glucose | 4,0 g |
| destilliertes Wasser | 1 l |
| pH | 7,0 |
| 72 Std. bei 30 °C. | |

(2) Zusammensetzung des Produktionsmediums

| | |
|---|---|
| Stärke | 10,0 g |
| Glucose | 10,0 g |
| Sojamehl | 15,0 g |
| $K_2HPO_4$ | 1,0 g |
| $MgSO_4$ | 1,0 g |
| NaCl | 3,0 g |
| $CuSO_4 \cdot 5H_2O$ | 0,007 g |
| $FeSO_4 \cdot 7H_2O$ | 0,001 g |
| $MnCl_2 \cdot 4H_2O$ | 0,008 g |
| $ZnSO_4 \cdot 7H_2O$ | 0,002 g |
| destilliertes Wasser | 1 l |
| pH | 7,0 |
| Aberntung nach 88-96 Std. | |

Beispiel 4

Isolierung des Rohgemisches der Anthracyclinverbindungen

Ca. 250 l Kulturlösung aus einem 300 l Fermenter wurden zentrifugiert.

a) 200 l Kulturfiltrat wurden mit NaOH auf pH 7,5 gestellt und 2 mal mit je 50 l Essigsäureäthylester extrahiert, dann die organische Phase im Vakuum eingeengt. Die dabei sich abscheidende wäßrige Phase wurde abgetrennt und 3 mal mit Essigester nachextrahiert. Die vereinigten organischen Phasen wurden im Vakuum zur Trockene eingedampft und der Rückstand in 650 ml Toluol gelöst, die Lösung 5 mal mit je 300 ml Natriumacetatpuffer pH 3,5 extrahiert, die Toluolphase im Vakuum eingedampft (roter öliger Rückstand, Fraktion A). Die vereinigten wäßrigen Phasen wurden mit 2 n NaOH auf pH 7,5 gebracht und 4 mal mit 400 ml Essigester extrahiert. Die abgetrennten Essigesterphasen wurden im Vakuum zur Trockene eingedampft (0,7 g tiefroter fester Rückstand : Fraktion B).

b) 9,5 kg festes Mycel wurden 2 mal mit je 50 l Aceton-Acetatpuffer pH 3,5 (10 : 1) extrahiert, die vereinigten Extrakte im Vakuum auf 12 l eingeengt (pH 3,5), das Konzentrat 3 mal mit 4 l Toluol gewaschen, die Toluolphasen vereinigt und im Vakuum zu einem öligen tiefroten Rückstand eingeengt. Die wäßrige Phase wurde mit Konz. NaOH auf pH 7,5 gestellt, 3 mal mit je 4 l Essigester extrahiert und die vereinigten Essigesterphasen im Vakuum eingedampft. Es verblieben 1,98 g eines tiefroten festen Rückstandes (Fraktion B).

c) das weitere Isolierungsverfahren ist aus den Schaubildern I und II ersichtlich.
Die Cytorhodine A, B, C, D, E, F und H und das 1 : 1-Gemisch G + I wurden durch wiederholte Chromatographie der an Glykosid angereicherten Fraktionen (Schaubild II) z. B. über Kieselgel oder Sephadex LH 20 oder an « reversed phase » Adsorbention oder DCCC (droplet counter current chromatography), oder durch Verteilung zwischen Toluol und Methanol/Wasser (1 : 1) isoliert. Die letzte Reinigungsstufe erfolgte mittels präparativer Dünnschicht- und Hochdruckflüssigkeitschromatographie (HPLC). Die HPLC erfolgte unter Verwendung einer Säule mit Microporasil® (Waters) oder Lichrosorb® Si 60 (Merck) und einer mobilen Phase der folgenden Zusammensetzung : Chloroform : Methanol : Essigsäure : Wasser : Triäthylamin im Verhältnis 68 : 20 : 10 : 2 : 0,01. Der Fluß wurde auf 0,5-1,5 ml pro Minute eingestellt und der Nachweis erfolgte mittels UV-Absorption bei 254, 260 bzw. 490 nm in einem Durchflußphotometer.

Beispiel 5

Isolierung der Verbindungen Cytorhodin A, B und H als Rohgemisch

1,0 g rohes Cytorhodingemisch, gewonnen durch Extraktion aus der Kulturlösung wie im Schema I beschrieben, wurde an 100 g Kieselgel 31 μ (Grace) mit dem Gemisch $CHCl_3$/Methanol/96 %ige Essigsäure/Wasser/Triäthylamin 80 : 5 : 5 : 1 : 0,01 in einer Glassäule 3 × 43 cm chromatographiert. Die Probe wurde in 7 ml der mobilen Phase gelöst, auf die mit dem Eluenten äquilibrierte Säule gegeben und in Fraktionen von 23 ml aufgefangen und mit Dünnschichtchromatographie auf DC-Fertigplatten Kieselgel 60 F 254 (Merck) mit dem System $CHCl_3$/Methanol/96 %ige Essigsäure/Wasser/Triethylamin 80 : 10 : 10 : 2 : 0,01 (System B) beurteilt :

| | | | |
|---|---|---|---|
| 1 - 139 | 238 mg | Gemisch der Verbindungen | C, D, E, F, G+I |
| 140 - 220 | 189 mg | " " " | A, B, H, M |
| 221 - 288 | 138 mg | " polarer Produkte | (Fraktion "Y") |

In analoger Weise wurden weitere Säulenchromatographien mit dem Rohprodukt Cytorhodin-Gemisch durchgeführt. Gleichartig zusammengesetzte Mischfraktionen wurden mittels HPLC in Stahlsäulen weiter aufgetrennt.

Beispiel 6

Isolierung der Einzelkomponenten Cytorhodin A, B und H durch Hochdruckflüssigkeitschromatographie (HPLC)

52,5 mg einer Mischfraktion mit Cytorhodin A, B und H wurden in 2 ml des Gemisches $CHCl_3$/MeOH/96 %ige Essigsäure/Wasser/Triäthylamin 80 : 10 : 10 : 2 : 0,01 gelöst und auf eine Stahlsäule (2,1 × 25 cm) gegeben, die unter Druck mit 40 g LiChrosorb® Si60 7 μ Merck, gefüllt und mit der obigen mobilen Phase äquilibriert worden war. Die Elution erfolgte mit einem Fluß von 5 ml/min und wurde mit einem Durchfluß-Spektralphotometer bei einer Wellenlänge von 490 nm verfolgt, wobei Fraktionen von 4 ml aufgefangen und nach Prüfung mit analytischer HPLC zusammengefaßt wurden :

| Fraktion | | Verbindungen | $R_F$ System A |
|---|---|---|---|
| 19 - 22 | 6 mg | Cytorhodin B | 0.33 |
| 23 - 27 | 7 mg | Mischfraktion Cytorhodin B + H + A | 0.3 |
| 28 - 37 | 15 mg | Cytorhodin A | 0.27 |

Zur Isolierung der Verbindung Cytorhodin H wurden die Mischfraktionen aus mehreren Läufen in gleicher Weise rechromatographiert.

Die reinen Einzelkomponenten aus mehreren chromatographischen Läufen wurden vereinigt und durch das folgende Verfahren nochmals gereinigt.

120 mg vereinigte Chargen von durch mehrfache Säulenchromatographie wie beschrieben isoliertem Cytorhodin A wurden in 30 ml Natrium-Acetatpuffer pH 3,5 gelöst, die Lösung mit 2 n NaOH auf pH 7,8 gestellt und 4 mal mit je 15 ml Essigsäureäthylester extrahiert, die vereinigten organischen Phasen wurden 1 mal mit 1 ml einer 0,001 molaren Lösung von EDTA (pH 7,5) und anschließend 2 mal mit je 2 ml Wasser gewaschen, über $NaSO_4$ getrocknet, filtriert und i. V. eingedampft. Der Rückstand wurde in wenig $CHCl_3$ gelöst, die Lösung durch eine Glasfritte abgesaugt und nach Zusatz von Heptan bis zur Trübung i. V. eingedampft.

| | | | | |
|---|---|---|---|---|
| **A:** | $^1$H-NMR | Abb. 1 | | |
| Absorptions-spektrum | a) 235 (4.62) | 253 (Sch) | 295 (3.87) | 4.95 (4.11) |
| | b) 235 (4.62) | 255 (Sch) | 296 (3.92) | 4.97 (4.17) |
| | c) 243 (4.63) | - | 280 (3.95), 305 (3.91), 575 u. 610 (4.15) | |

$C_{60}H_{88}N_2O_{20}$, M ber. : 1156 (FAB-MS bestätigt)

| | | | | |
|---|---|---|---|---|
| **B:** | $^1$H-NMR | Abb. 2 | | |
| Absorptions-spektrum | a) 235 (4.52) | 252 (Sch) | 297 (3.83) | 4.95 (3.94) |
| | b) 236 (4.52) | 253 (Sch) | 295 (3.89) | 4.95 (4.03) |
| | c) 242 (4.40) | - | 280 (3.89), 305 (3,69), 610 (3.83) | |

$C_{60}H_{86}N_2O_{21}$, M ber. : 1170 (FAB-MS bestätigt)

Beispiel 7

Isolierung von Cytorhodin F

2 g rohes Cytorhodin wurden in 50 ml eines Gemisches von Chloroform/Methanol/Eisessig/Wasser 80 : 10 : 10 : 2 (System A) gelöst und auf eine Säule mit Kieselgel 60 « Merck », 15 — 40μ, 2,75 × 42 cm,

aufgeschlämmt in System A, aufgetragen. Als Elutionsmittel wurde System A, das 0,01 % Na-Heptansulfonat enthält, benutzt. Nach einem Vorlauf von 1,1 l wurden Fraktionen von je 25 ml aufgefangen und dünnschichtchromatographisch geprüft (Kieselgel 60 $F_{254}$ (Merck), System A). Die aktiven Komponenten wurden in folgenden Fraktionen eluiert:

| Fraktion | Cytorhodin | | $R_F$ System A |
|---|---|---|---|
| 40 - 44 | F | 20 mg | 0,63 |
| 45 - 49 | F + D | 60 mg | |
| 50 - 76 | D | 263 mg | 0,53 |
| 77 - 93 | D + C | 145 mg | |
| 94 - 126 | C | 260 mg | 0,44 |
| 127 - 153 | C, P+V, G+I | 135 mg | |

Nachlauf « X » (durch Waschen der Säule mit Methanol)

Die vereinigten Fraktionen werden mit gesättigter wäßriger Lösung von $Na_2HPO_4$ bis zur Abscheidung der Chloroformphase versetzt, die Chloroformphase mit je einem Volumen 5 %iger $Na_2HPO_4$-Lösung und dann mit Wasser gewaschen, über wasserfreiem Natriumsulfat getrocknet, im Vakuum eingeengt und mit Petroläther oder Hexan gefällt.

F: $^1$H-NMR: Abb. 3

Absorptionsspektrum;

| | | | |
|---|---|---|---|
| a) 235 (4.55) | 254 (Sch) | 295 (3.79) | 495 (4.04) |
| b) 235 (4.55) | 255 (Sch) | 295 (3.79) | 495 (4.04) |
| c) 243 (4.49) | – | 280 (Sch, 382) | 580, 610 (3.95) |

$C_{60}H_{80}N_2O_{20}$, M ber. 1148

## Beispiel 8

Fraktionierung des Cytorhodins-Rohgemisches durch Verteilung zwischen Toluol und Methanol-Wasser

Eine einfache schnelle Auftrennung des Cytorhodin-Rohgemisches in einen weniger polaren Anteil (Komplex I) und einen stärker polaren Anteil (Komplex II) wurde durch Verteilung zwischen Toluol und Methanol/Wasser erreicht.

83 g rohes Cytorhodin-Gemisch, gewonnen durch Extraktion von Mycel, werden in 500 ml Methanol-Wasser (1 : 1) gelöst und 5 mal mit jeweils 500 ml Toluol extrahiert. Die vereinigten Toluolphasen ergaben nach dem Eindampfen im Vakuum 32 g eines Cytorhodingemisches (Komplex I) mit überwiegend schwächer polaren Komponenten (vor allem Cytorhodine F, D, C, P, V, X), die Methanol-Wasser-Phase dagegen 47 g eines Cytorhodingemisches (Komplex II) mit vorwiegend stärker polaren Komponenten (vor allem die Cytorhodine B, H, A, M und Fraktion Y, wobei die Fraktion Y unter anderem enthält die Cytorhodine M, S, N + O und W).

Die so gewonnenen Produkte erweisen sich als günstige Ausgangsprodukte für die weitere Auftrennung und Isolierung der Cytorhodine durch Säulenchromatographie oder DCC-Chromatographie (droplet counter current chromatography) oder durch Kombination von beiden Verfahren.

## Beispiel 9

Anreicherung der Verbindungen Cytorhodin A und N + O durch Säulenchromatographie an modifiziertem Kieselgel

10 g rohes polares Cytorhodingemisch (Komplex II), gewonnen durch Verteilung zwischen Toluol-Methanol-Wasser wurden in 30 ml des Gemisches (mobile Phase) $CHCl_3/H_2O/MeOH$ 130 : 40 : 40 (Unter-Phase) gelöst und auf eine Glassäule 5,5 × 95 cm mit ca. 870 g modifiziertem Kieselgel 31 μ (Grace)

gegeben und mit obigem Lösungsmittelgemisch chromatographiert. Das verwendete Kieselgel war mit 2 N HCl metallfrei gewaschen und nach Auswaschen der Säure in wäßriger Suspension mit 5 N NaOH behandelt worden, bis sich ein pH von 7,6 eingestellt hatte. Nach Dekantieren wurde das modifizierte Kieselgel im Trockenschrank bei 130 °C getrocknet, gesiebt und mit der mobilen Phase in die Säule eingeschlämmt. Die Chromatographie erfolgte mit einem Fluß von ca. 100 ml/h zunächst mit 5,5 l der oben angegebenen mobilen Phase, dann mit 5,3 l des Gemisches $CHCl_3/H_2O/MeOH$ 130 : 40 : 50 bei Fraktionen von ca. 15 ml. Nach Beurteilung durch DC wurden die eluierten Fraktionen in geeigneter Weise zu Gruppen zusammengefaßt und im Vakuum eingedampft. Man erhielt so nach einem Vorlauf von ca. 2 Liter :

| Fraktion | l | g | Verbindungen |
|---|---|---|---|
| 1 - 52 | 0,750 | 0,63 | Gemisch unpolarer Verbindungen (u.a. C u. D) |
| 53 - 343 | 4,1 | 2,64 | Gemisch von Verbindungen mittlerer Polarität (u.a. V, G+I bis B) |
| 344-400 | 0,9 | 1,7 | Gemisch von Verbindungen höherer Polarität (u.a. B, A) |
| 401-430 | 0,3 | 0,56 | ca. 40 % A, ca. 35 % N+O |
| 431-560 | 1,1 | 1,3 | Gemisch sehr polarer Verbindungen (u.a. A, N+O, W) |

Mit 1 l MeOH wurde von der Säule ein Gemisch der polaren bis sehr polaren Gruppen heruntergewaschen.

## Beispiel 10

Fraktionierung eines angereicherten polaren Cytorhodin-Gemisches durch Tropfengegenstrom-Chromatographie (DCCC)

5 g eines durch Verteilung zwischen Toluol und Methanol/Wasser gewonnenen, an stärker polaren Komponenten angereicherten Cytorhodin-Gemisches wurden durch DCCC mit dem DCC-Chromatograph 670 (Büchi) aufgetrennt. Dazu wurde die Probe in 20 ml der Unterphase des heterogenen äquilibrierten Lösungsmittelgemisches $CHCl_3$/Wasser/Methanol/n-Propanol 45 : 90 : 120 : 5 (nach Äquilibrierung mit 1 % 96 %iger Essigsäure versetzt) gelöst und eingepumpt. Bei einem Fluß von ca. 40 ml/h und einem Druck von 5 bis 10 bar wurden nach einem Vorlauf von 200 ml ca. 3 l der Unterphase innerhalb von 3 Tagen durch die mit der stationären Phase (mit 1 % 96 %iger Essigsäure versetzte Oberphase des obigen Gemisches) gefüllten Glasröhren (ID, 2,7 mm) gepumpt (« descending mode »). Die ausgetragene mobile Phase wurde in Fraktionen von 10 ml aufgefangen. Nach Beurteilung durch DC und analytische HPLC wurden zusammengehörende Fraktionen zusammengefaßt und im Vakuum eingedampft. Man erhielt :

| Fraktion | mg | Komponenten |
|---|---|---|
| Vorlauf | 800 (ölig) | kaum verändertes Ausgangsgemisch, unpolare Anteile |
| 1 - 21 | 660 | Mischung von V, L, B |
| 22 - 27 | 450 | B mit geringen Mengen weniger polaren Anteilen (V, L) |
| 28 - 33 | 500 | B (mit weniger polaren Verunreinigungen) |
| 34 - 55 | 230 | A, M und L |
| 56 - 63 | 200 | A, M |
| 64 - 75 | 420 | A, M, stärker polare Komponenten |
| 76 - 100 | 1300 | Fraktion Y (mit N+O, W) |

Beispiel 11

Isolierung der Cytorhodine A und N + O aus einem stark angereicherten Gemisch durch « reversed phase » HPLC

50 mg einer Mischfraktion mit ca. 40 % Cytorhodin A und ca. 35 % Cytorhodine N + O wurden in 1,5 ml der mobilen Phase $CHCl_3$/MeOH/10 % Ammoniumacetat in $H_2O$ 150 : 1050 : 375 gelöst und in einer Stahlsäule 1,6 × 25 cm an 30 g LiChrosorb® RP-18, 10 µ (Merck) bei einem Fluß von 2 ml/min. chromatographiert. Mit einem Druckflußphotometer wurde bei der Wellenlänge 490 nm der Verlauf der Elution verfolgt. Die Fraktionen von 4 ml wurden nach Beurteilung durch analytische HPLC (ebenfalls an einer Stahlsäule gefüllt mit 10 µ LiChrosorb® RP-18 (Merck)) zusammengefaßt und aufgearbeitet. Dazu wurde mit der Hälfte des Volumens an Wasser verdünnt und $CHCl_3$ bis zur Phasentrennung zugesetzt und die abgetrennte $CHCl_3$-Phase mit $H_2O$ gewaschen, über $Na_2SO_4$ getrocknet und im Vakuum eingedampft. Erhalten wurden :

| Fraktion | | | RF System C |
|---|---|---|---|
| 15 - 17 | ca. 5 mg | angereichertes Cytorhodin M | |
| 26 - 30 | 22,5 mg | Cytorhodin N+O | 0.30 |
| 35 - 40 | 10,4 mg | Cytorhodin A | 0.41 |

System C : $CHCl_3$/Methanol/99 % Essigsäure/Wasser 75 : 15 : 10 : 2.

Cytorhodin N+O: 1H-NMR Abb. 4

| | | | | | |
|---|---|---|---|---|---|
| Absorptions- | a) | 235 (4.56) 25 | (Sch) | 293 (3.67) | 495 (4.04) |
| spektrum | b) | 234 (4.59) 25 | (Sch) | 293 (3.68) | 495 (4.15) |
| | c) | 240 (4.51) | | 283 (3.81) | 575 (4.0) |
| | | | | | 615 (3.98) |

$C_{60}H_{88}N_2O_{21}$, M ber. 1172 (FAB-MA bestätigt).

Das isolierte Produkt ist ein 1 : 1-Gemisch der beiden strukturisomeren Komponenten N und O. Dies geht aus dem 1H-NMR-Spektrum, Abbauversuchen (Hydrogenolyse) und Bestimmung der einzelnen Zuckerbausteine nach Hydrogenolyse und Totalhydrolyse hervor. Die Komponente Cytorhodin O ist literaturbekannt (H. Brockmann, H. Greve, Tetrahedron Letters 1975 (831-834)).

Beispiel 12

Isolierung der Einzelkomponente Cytorhodin P durch (Mittel) druckchromatographie und präparative Dünnschichtchromatographie

6 g rohes Cytorhodingemisch, gewonnen durch Extraktion aus der Kulturlösung wie in Schema I beschrieben, wurden an 620 g Kieselgel 31 µ (Grace) in zwei radial komprimierten Silica-Patronen (Waters, PrepLC/System 500®) mit dem Laufmittelgemisch $CHCl_3$/Methanol/96 % Essigsäure/Wasser = 80/10/10/2 chromatographiert. Die Probe wurde in 50 ml der mobilen Phase auf die äquilibrierte Säule gegeben und bei einem Fluß von 35 ml/Min in Fraktionen zu 23 ml aufgetrennt. Die Beurteilung erfolgte dünnschichtchromatographisch und mit Hilfe der analytischen HPLC.

In Fraktion 41-56 fielen 220 mg Cytorhodin P in einer Reinheit von 88 % an ($R_f$ 0.42 im System A).

40 mg dieser Probe wurden durch präparative Dünnschichtchromatographie (Merck, DC-Fertigplatten Kieselgel 60) mit dem System A weiter gereinigt.

Die Elution der Kieselgelbande erfolgte mit $CHCl_3$/Methanol 1/1. Die Elutionslösung wurde mit wäßriger Dinatriumhydrogenphosphat-Lösung neutralisiert und mit Wasser bis zur Abtrennung der $CHCl_3$-Phase versetzt. Die Chloroformphase wurde separiert, einmal mit wenig Wasser gewaschen, über $Na_2SO_4$ getrocknet und eingedampft. Nach Aufnehmen in wenig $CHCl_3$, Fällung mit der 10-fachen Volumenmenge Petrolether und Trocknung des Niederschlages wurden 15 mg reines P erhalten.

P: $^1$H-NMR Abb. 5

Absorptionsspektrum (nm):

| | | | | |
|---|---|---|---|---|
| a) | 235 (4,63) | 254 (SCH 4,33) | 293 (3,90) | 494 (4,13) |
| b) | 235 (4,60) | 254 (SCH 4,31) | 293 (3,85) - | 494 (4,12) |
| c) | 244 (4,33) | - | | 570 (3,59) |
| | | | | 620 (3,71) |

$C_{60}H_{82}N_2O_{21}$, M ber. 1166 (FAB-MS bestätigt)

## Beispiel 13

Isolierung der Einzelkomponente Cytorhodin V durch Druckchromatographie und präparative Dünnschichtchromatographie

7 g rohes Cytorhodingemisch, gewonnen durch Extraktion aus der Kulturlösung wie in Schema I beschrieben, wurden an 620 g Kieselgel 31 µ (Grace) in zwei radial komprimierten Silica-Patronen (Waters, PrepLC/System 500®) mit dem Laufmittelgemisch $CHCl_3$/Methanol/96 % Essigsäure/Wasser = 80/10/10/2 chromatographiert. Die Probe wurde in 65 ml der mobilen Phase auf die äquilibrierte Säule gegeben und bei einem Fluß von 25 ml/Min in Fraktionen zu 23 ml aufgetrennt. Die Beurteilung erfolgte dünnschichtchromatographisch und mit Hilfe der analytischen HPLC.

In Fraktion 29-34 fielen 195 mg Cytorhodin V in einer Reinheit von 80 % an ($R_f$ 0.38 im System A).

60 mg dieser Probe wurden durch präparative Dünnschichtchromatographie (Merck, DC-Fertigplatten Kieselgel 60) mit dem System A weiter gereinigt.

Die Elution der Kieselgelbande und die Gewinnung von reinem Cytorhodin V erfolgte wie unter Beispiel 14 beschrieben. Es wurden 16 mg reines V erhalten.

V: $^1$H-NMR Abb. 6

Absorptionsspektrum (nm):

| | | | | |
|---|---|---|---|---|
| a) | 235 (4,72) | 254 (SCH 4,48) | 290 (4,04) | 495 (4,20) |
| b) | 235 (4,73) | 254 (SCH 4,48) | 290 (4,06) | 494 (4,24) |
| c) | 244 (4,61) | 268 (4,63) | - | 570 (4,22) |
| | | | | 601 (4,19) |

$C_{60}H_{88}N_2O_{20}$, M ber. 1152 (FAB-MS bestätigt)

## Beispiel 14

Isolierung von Cytorhodin W durch präparative Hochdruckflüssigkeitschromatographie (HPLC)

80 mg der polaren Fraktion Y wurden mit 2 ml der mobilen Phase $CHCl_3$/Methanol/96 % Essigsäure/Triäthylamin 80 : 10 : 10 : 0,01 (wassergesättigt) gelöst und auf eine 3,2 × 25 cm Stahlsäule gepackt mit 110 g 7 µ LiChrosorb® Si 60 (Merck) gegeben und unter Druck bei einem Fluß von 8 ml/Min chromatographiert. Fraktionen von 4 ml wurden aufgefangen und mit analytischer HPLC beurteilt, vereinigt und in der üblichen Weise aufgearbeitet. Neben anderen nicht identifizierten Komponenten wurden 1,5 mg der Verbindung Cytorhodin W isoliert, die im System C auf den üblichen DC-Fertigplatten Kieselgel 60 (Merck) den RF-Wert 0,23 zeigte.

W:

| | | | | | |
|---|---|---|---|---|---|
| Absorptions- | a) | 235 (4.53) | 253 (Sch) | 293 (3.64) | 495 (4.01) |
| spektrum | b) | 235 (4.57) | 253 (Sch) | 293 (3.65) | 495 (4.13) |
| | c) | 241 (4.49) | - | 282 (3.78) | 575 (3.81) |
| | | | | | 610 (3.96) |

$C_{60}H_{88}N_2O_{22}$, M ber. 1184

Die Identifizierung der Komponenten in den vorstehenden Beispielen erfolgte unter den anschließend beschriebenen Meßbedingungen :

Die Protonen-Resonanzspektren ($^1$H-NMR-Spektren) wurden auf einen HX-270 BRUKER Fourier-Transform Kernresonanzspektrometer bei 270 MHZ gemessen. Die Konzentrationen betrugen 2-4 mg/0,5 mg 99.8 % $CDCl_3$ ; die Lösungen wurden sofort nach Herstellung mit 0,1 ml 5 %iger $Na_2CO_3$ 99,5 % $D_2O$ geschüttelt.

Die in den Abbildungen mit einem Stern versehenen Signale rühren her von niedermolekularen Verunreinigungen in %o Bereich und von Lösungsmittelresten.

Die Massenspektren wurden auf dem Massenspektrometer MS-902 S, AEI, unter Verwendung einer FAB-(Fast-Atom-Bombardment-)Ionenquelle gemessen. Die Substanzen wurden in einer Matrix von Thioglycerin in die Ionenquelle eingebracht, teilweise unter Zasatz von Ammoniumchlorid.

Die Absorptionsspektren wurden im Bereich 200-700 nm gemessen in :

a) Wasser-Methanol 1 : 9
b) 10 % 1n HCl in Methanol
c) 10 % 1n NaCH in Methanol

Die Substanzkonzentration betrug 10-30 mg/l ; angegeben werden die Absorptionsmaxima in nm und die molaren Extinktionskoeffizienten (log ε).

Ermittlung der zytotoxischen Aktivität

Die Bestimmung der zytostatischen Wirksamkeit der hier beschriebenen Verbindungen erfolgte an L1210 Leukämiezellen der Maus. Im einzelnen wurden folgende Testsysteme verwendet :

a) Proliferationsassay

Bei dieser Methode wird in vitro nach Inkubation der Zellen mit unterschiedlichen Konzentrationen der Testsubstanz ermittelt, inwieweit die Zellen radioaktive DNA-Vorläufer (z. B. C14 markiertes Thymidin) einbauen können. Unbehandelte L1210 Zellen werden den gleichen Testbedingungen unterworfen und dienen als Kontrolle. Im folgenden ist die Methode kurz beschrieben :

L1210 Zellen in exponentieller Wachstumsphase ($5 \times 10^3$/ml in RPMI 1640) werden in einer Mikrotiterplatte 72 Stunden mit unterschiedlichen Konzentrationen der Testsubstanz inkubiert (37 °C, 5 % $CO_2$, 95 % relative Luftfeuchte). Kontrollen bestehen aus Zellen, die lediglich mit frischen Medium inkubiert werden. Alle Bestimmungen werden als 4-fach Bestimmungen durchgeführt. Nach 65 Stunden werden 50 µl C114 Thymidin (1,5 µCi/ml) zugegeben, um die DNA der Zelle radioaktiv zu markieren. Nach 7 Stunden Inkubation werden die Zellen abgesaugt, die DNA mit 5 %-iger Trichloressigsäure gefällt und nacheinander mit Wasser bzw. Methanol gewaschen.

Nach Trocknung bei 50 °C wird die in die DNA eingebaute Radioaktivität nach Zugabe von 5 ml Szintillationsflüssigkeit ermittelt.

Die Ergebnisse werden angegeben als Verhältnis des Szintillationsindex nach Inkubation mit der Testsubstanz in Prozent der unbehandelten Kontrolle. Aus den so erhaltenen Meßwerten wird die Dosiswirkungskurve ermittelt und graphisch die $IC_{50}$, d. h. die Konzentration, die unter Testbedingungen den Einbau von radioaktivem Thymidin um 50 % gegenüber der Kontrolle erniedrigt, ermittelt. Die $IC_{50}$ Werte der hier beschriebenen Verbindungen im Vergleich zu Adriamycin (ADM) werden in der Tabelle 1 zusammengefaßt.

b) Koloniebildung von L1210 Leukämiezellen in soft agar. Diese Methode dient zum Nachweis eines Einflusses der Testsubstanzen auf das Wachstumsverhalten der Zellen über mehrere Generationen (bei einer Zellzykluszeit von 10-12 Stunden werden in der Testzeit von 7 Tagen ca. 14 aufeinanderfolgende Generationen Beobachtet).

Zytostatisch wirksame Substanzen bewirken in diesem Test eine Reduktion der zu beobachtenden Koloniezahl gegenüber einer unbehandelten Kontrolle. Im einzelnen wird der Test wie folgt durchgeführt :

500 Leukämiezellen pro Platte werden mit unterschiedlichen Konzentrationen von Testsubstanz 1 Stunde bei 37 °C inkubiert. Anschließend werden die Zellen zweimal mit McCoy5a Medium gewaschen und schließlich in Petrischalen nach Zusatz von 0.3 % Agar ausgegossen. Kontrollen werden lediglich mit frischem Medium inkubiert. Anstelle der 1stündigen Inkubation werden in manchen Fällen unterschiedliche Konzentrationen der Testsubstanz der oberen Agarschicht zugemischt, um so eine kontinuierliche Exposition der Zellen über die gesamte Inkubationszeit zu erreichen. Nach Erstarren des Agars werden die Platten im Brutschrank 7 Tage bei 37 °C inkubiert (5 % $CO_2$, 95 % relative Luftfeuchtigkeit). Anschließend wird die Anzahl der entstandenen Kolonien mit einem Durckmesser 60 µ gezählt. Die Ergebnisse werden angegeben als Koloniezahl in behandelten Agarplatten in Prozent der unbehandelten Kontrolle. Aus der so erhaltenen Dosiswirkungskurve wird die $IC_{50}$ als Maß für die Wirksamkeit der Substanz ermittelt. Die Ergebnisse für die hier beschriebenen Verbindungen im Vergleich zu Adriamycin sind in der Tabelle 1 zusammengefaßt.

Tabelle 1

| | Proliferationstest | Koloniebildungstest | |
|---|---|---|---|
| | $IC_{50}$ (µg/ml) | $IC_{50}$ (µg/ml) | |
| | | 7 Tage Inkubation | 1 Stunde Inkubation |
| ADM | $6,0 \times 10^{-3}$ | $2,2 \times 10^{-2}$ | $4,4 \times 10^{-2}$ |
| Cytorhodin B | $2,8 \times 10^{-3}$ | $3,4 \times 10^{-3}$ | $1,2 \times 10^{-3}$ |
| " A | $1,1 \times 10^{-3}$ | $3,0 \times 10^{-3}$ | $1,3 \times 10^{-3}$ |
| " F | $2,4 \times 10^{-3}$ | $2,8 \times 10^{-2}$ | $4,2 \times 10^{-2}$ |
| P | $2,6 \times 10^{-3}$ | $3,2 \times 10^{-4}$ | $2,8 \times 10^{-3}$ |
| V | $2,9 \times 10^{-3}$ | $2,7 \times 10^{-4}$ | $2 \times 10^{-3}$ |
| N + O | $5 \times 10^{-3}$ | $2,5 \times 10^{-3}$ | $4,5 \times 10^{-2}$ |

Tabelle 2

Charakteristika von Streptomyces Y-11472

a) Morphologie

Abteilung Spirales. Reife, mäßig lange Sporenketten mit wenigstens 10 bis 50 Sporen pro Kette. Diese Morphologie zeigt sich auf Hefe-Malz-Agar, auf Hafermehl-Agar, auf Salz-Stärke-Agar und Glycerin-Asparagin-Agar.

b) Kulturcharakteristika auf

| Medium | Wachstum | Rückseite | Luftmycel | Lösliches Pigment |
|---|---|---|---|---|
| 1. Hefemalz-Agar | gut, runzlig, trocken | rötlich-violett | gut, baumwollartig-samtig, gelb-rosa | braun-rosa |
| 2. Hafermehl-Agar | gut, flach, trocken | blaßrosa | gut, puderig, gelb-rosa | blaßrosa |
| 3. Mineralsalze-Stärke-Agar | mäßig, flach, trocken | farblos bis blaß gelb-rosa | gut, baumwollartig-samtig, leicht | blaß gelb-rosa |
| 4. Glycerin-Asparagin-Agar | mäßig, runzlig, trocken | rot-violett | mäßig, puderig, grau-rosa | blaß rot-violett |
| 5. Kartoffel-Glucose-Agar | gut, erhaben, trocken | braun | gut, samtig, blaß gelb-weiß | violett |
| 6. Pepton-Hefe-Eisen-Agar | gut, erhaben, feucht | farblos bis blaß gelb | entfällt | rot-braun |
| 7. Tyrosin-Agar | gut, erhöht, trocken | braun | gut, baumwollartig-samtig, leicht, grau-rosa | rot-braun |
| 8. Czapeck's-Agar | gut, runzlig, trocken | purpurrot | mäßig, baumwollartig-samtig, blaß gelb-rosa | leicht purpurrot |

c) Physiologische Eigenschaften

1. Temperaturbereich für das Wachstum: wächst auf Hefe-Malz-Agar in einem Temperaturbereich von 24-40 °C mit optimalem Wachstum bei 30 °C.

| | |
|---|---|
| 2. Nitratreduzierung: | positiv |
| 3. Melaninbildung: | positiv |
| 4. Gelatineverwertung: | positiv |
| 5. Stärkehydrolyse: | positiv |
| 6. Tyrosinhydrolyse: | positiv |
| 7. Natriumchloridtoleranz: | > 7,0 % aber < 10,0 % |
| 8. Caseinhydrolyse: | positiv |
| 9. Gelatineverflüssigung: | positiv |
| 10. Ureaseherstellung: | positiv |
| 11. Streptomycinhemmung: | Hemmung bei 12,5 µg per ml |
| 12. pH-Empfindlichkeit: | |

Das Substrat-Mycel und das diffusionsfähige Pigment sind pH-empfindlich. Violett (purpurrot) unter alkalischen Bedingungen, rot (rosa) unter sauren Bedingungen.

d) Verwertung von Kohlenstoff

D-Glucose, L-Arabinose, Sucrose, D-Xylose, I-Inositol, D-Mannit, D-Fructose, Rhamnose, Raffinose, Galactose, Salicin, Maltose, Cellobiose, Ribose, Na-Glutamat und Sorbit wurden zum Anwachsen verwendet. Die Verwertung von Cellulose ist nicht eindeutig.

**Patentansprüche** (für die Vertragsstaaten: BE, CH, LI, DE, FR, GB, IT, LU, NL, SE)

1. Verbindungen der Formel

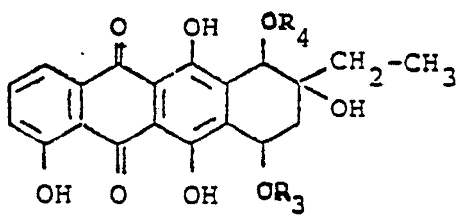

worin, falls $R_4$ für die Zuckerkombination Roa-Rod-Rod steht, $R_3$ die Bedeutung Roa-Rod-Rod, Roa-dF-Cin A oder Roa-Rod-Acu hat, oder falls $R_4$ für die Zuckerkombination Roa-Rod-Acu steht, $R_3$ die Bedeutung Roa-Rod-Acu oder Roa-dF-Cin A hat, oder, falls $R_4$ für die Zuckerkombination Roa-dF-Rod steht, $R_3$ die Bedeutung Roa-Rod-Rod oder Roa-dF-Rod hat, wobei

| | |
|---|---|
| Roa | Rhodosamin |
| dF | Desoxyfucose |
| Rod | Rhodinose |
| Acu | Aculose, und |
| Cin A | Cinerulose A |

bedeutet,
sowie die physiologisch unbedenkliche Säureadditionssalze.

2. Eine Verbindung gemäß Anspruch 1, wobei $R_3$ die Zuckerkombination Roa-dF-Cin A und $R_4$ die Zuckerkombination Roa-Rod-Rod darstellt, sowie deren physiologisch unbedenkliche Säureadditionssalze.

3. Eine Verbindung gemäß Anspruch 1, wobei $R_3$ und $R_4$ die Zuckerkombination Roa-Rod-Rod darstellen, sowie deren physiologisch unbedenkliche Säureadditionssalze.

4. Eine Verbindung gemäß Anspruch 1, wobei $R_3$ die Zuckerkombination Roa-Rod-Acu und $R_4$ die Zuckerkombination Roa-Rod-Rod darstellt, sowie deren physiologisch unbedenkliche Säureadditionssalze.

5. Eine Verbindung gemäß Anspruch 1, wobei $R_3$ und $R_4$ die Zuckerkombination Roa-Rod-Acu darstellen, sowie deren physiologisch unbedenkliche Säureadditionssalze.

6. Eine Verbindung gemäß Anspruch 1, wobei $R_3$ die Zuckerkombination Roa-dF-Cin A und $R_4$ die Zuckerkombination Roa-Rod-Acu darstellt, sowie deren physiologisch unbedenkliche Säureadditionssalze.

7. Eine Verbindung gemäß Anspruch 1, wobei $R_3$ die Zuckerkombination Roa-Rod-Rod und $R_4$ die Zuckerkombination Roa-dF-Rod darstellt, sowie deren physiologisch unbedenkliche Säureadditionssalze.

8. Eine Verbindung gemäß Anspruch 1, wobei $R_3$ und $R_4$ die Zuckerkombination Roa-dF-Rod darstellen, sowie deren physiologisch unbedenkliche Säureadditionssalze.

9. Verfahren zur Herstellung von Verbindungen gemäß Anspruch 1, dadurch gekennzeichnet, daß der Mikroorganismus Streptomyces purpurascens Y-11472 (DSM 2658) auf übliche Weise unter aeroben Bedingungen in Gegenwart eines Nährmediums fermentiert wird, und die gebildeten Verbindungen aus der Kulturflüssigkeit und dem Mycel durch Extraktion mit organischen Lösungsmitteln und anschließende Chromatographie oder Verteilung in üblicher Weise isoliert werden.

10. Arzneimittel, gekennzeichnet durch einen Gehalt an einer Verbindung gemäß Anspruch 1.

11. Verwendung einer Verbindung gemäß Anspruch 1 zur Herstellung eines Arzneimittels mit cytostatischer Wirkung.

12. Der Mikroorganismus Streptomyces purpurascens Y-11472 (DSM 2658).

**Patentansprüche** (für den Vertragsstaat AT)

1. Verfahren zur Herstellung von Verbindungen der Formel I

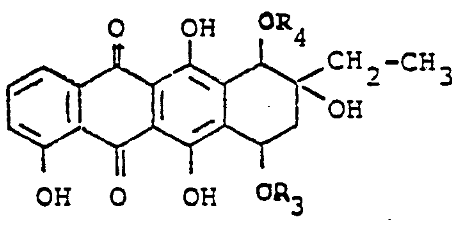

worin, falls $R_4$ für die Zuckerkombination Roa-Rod-Rod steht, $R_3$ die Bedeutung Roa-Rod-Rod, Roa-dF-Cin A oder Roa-Rod-Acu hat, oder falls $R_4$ für die Zuckerkombination Roa-Rod-Acu steht, $R_3$ die Bedeutung Roa-Rod-Acu oder Roa-dF-Cin A hat, oder, falls $R_4$ für die Zuckerkombination Roa-dF-Rod steht, $R_3$ die Bedeutung Roa-Rod-Rod oder Roa-dF-Rod hat, wobei

| | |
|---|---|
| Roa | Rhodosamin |
| dF | Desoxyfucose |
| Rod | Rhodinose |
| Acu | Aculose, und |
| Cin A | Cinerulose A |

bedeutet,

sowie deren physiologisch unbedenklichen Säureadditionssalze, dadurch gekennzeichnet, daß der Mikroorganismus Streptomyces purpurascens Y-11472 (DSM 2658) auf übliche Weise unter aeroben Bedingungen in Gegenwart eines Nährmediums fermentiert wird, und die gebildeten Verbindungen aus der Kulturflüssigkeit und dem Mycel durch Extraktion mit organischen Lösungsmitteln und anschließende Chromatographie oder Verteilung in üblicher Weise isoliert werden.

2. Verfahren gemäß Anspruch 1, dadurch gekennzeichnet, daß der Mikroorganismus Streptomyces purpurascens in einem Kohlenstoff- und Stickstoffquellen sowie anorganische Nährsalze sowie Spurenelemente enthaltenden Nährmedium bei Temperaturen von 24-40 °C und einem pH von 6,5-8,5 fermentiert wird, die gebildeten Verbindungen aus der Kulturflüssigkeit mit Äthylacetat oder Chloroform und aus dem Mycel zunächst mit wäßrigem Aceton, aus der abgetrennten wäßrigen Phase sodann mit Äthylacetat extrahiert werden, die vereinigten Äthylacetatextrakte konzentriert und mit Benzol oder Toluol aufgenommen, die erhaltene Lösung mit einem Acetatpuffer bei einem pH-Wert von 3,5 extrahiert wird und aus der erhaltenen Lösung durch Chromatographie oder Verteilung die Verbindungen der Formel I isoliert werden.

3. Verfahren gemäß Anspruch 1 und 2, dadurch gekennzeichnet, daß die chromatographische Isolierung der Verbindungen der Formel I durch Kieselgel-Säulen, « reversed phase »-Adsorbentien, Tropfen-Gegenstrom-Chromatographie oder Verteilung und anschließende Dünnschicht- oder Hochdruckflüssigkeitschromatographie erfolgt.

4. Verfahren gemäß Anspruch 1, wobei $R_3$ die Zuckerkombination Roa-dF-Cin A und $R_4$ die Zuckerkombination Roa-Rod-Rod darstellt.

5. Verfahren gemäß Anspruch 1, wobei $R_3$ und $R_4$ die Zuckerkombination Roa-Rod-Rod darstellen.

6. Verfahren gemäß Anspruch 1, wobei $R_3$ die Zuckerkombination Roa-Rod-Acu und $R_4$ die Zuckerkombination Roa-Rod-Rod darstellt.

7. Verfahren gemäß Anspruch 1, wobei $R_3$ und $R_4$ Zuckerkombination Roa-Rod-Acu darstellen.

8. Verfahren gemäß Anspruch 1, wobei $R_3$ die Zuckerkombination Roa-dF-Cin A und $R_4$ die Zuckerkombination Roa-Rod-Acu darstellt.

9. Verfahren gemäß Anspruch 1, wobei $R_3$ die Zuckerkombination Roa-Rod-Rod und $R_4$ die Zuckerkombination Roa-dF-Rod darstellt.

10. Verfahren gemäß Anspruch 1, wobei $R_3$ und $R_4$ die Zuckerkombination Roa-dF-Rod darstellen.

11. Verwendung einer Verbindung gemäß Anspruch 1 zur Herstellung eines Arzneimittels mit cytostatischer Wirkung.

**Claims** (for the Contracting States : BE, CH, LI, DE, FR, GB, LU, NL, SE)

1. A compound of the formula

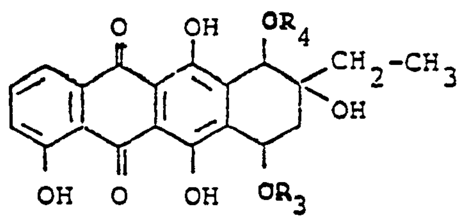

(I)

in which $R_3$ has the meaning Roa-Rod-Rod, Rad-dF-Cin A or Roa-Rod-Acu if $R_4$ represents the sugar combination Roa-Rod-Rod, or $R_3$ has the meaning Roa-Rod-Acu or Roa-dF-Cin A if $R_4$ represents the sugar combination Roa-Rod-Acu, or $R_3$ has the meaning Roa-Rod-Rod or Roa-dF-Rod if $R_4$ represents the sugar combination Roa-dF-Rod,
in which
Roa denotes rhodosamine,
dF denotes deoxyfucose,
Rod denotes rhodinose,
Acu denotes aculose, and
Cin A denotes cinerulose A,
and the physiologically acceptable acid addition salts.

2. A compound as claimed in claim 1, wherein $R_3$ represents the sugar combination Roa-dF-Cin A, and $R_4$ represents the sugar combination Roa-Rod-Rod, and its physiologically acceptable acid addition salts.

3. A compound as claimed in claim 1, wherein $R_3$ and $R_4$ represent the sugar combination Roa-Rod-Rod, and its physiologically acceptable acid addition salts.

4. A compound as claimed in claim 1, wherein $R_3$ represents the sugar combination Roa-Rod-Acu, and $R_4$ represents the sugar combination Roa-Rod-Rod, and its physiologically acceptable acid addition salts.

5. A compound as claimed in claim 1, wherein $R_3$ and $R_4$ represent the sugar combination Roa-Rod-Acu, and its physiologically acceptable acid addition salts.

6. A compound as claimed in claim 1, wherein $R_3$ represents the sugar combination Roa-dF-Cin A, and $R_4$ represents the sugar combination, Roa-Rod-Acu, and its physiologically acceptable acid addition salts.

7. A compound as claimed in claim 1, wherein $R_3$ represents the sugar combination Roa-Rod-Rod and $R_4$ represents the sugar combination Roa-dF-Rod, and its physiologically acceptable acid addition salts.

8. A compound as claimed in claim 1, wherein $R_3$ and $R_4$ represent the sugar combination Roa-dF-Rod, and its physiologically acceptable acid addition salts.

9. A process for the preparation of compounds as claimed in claim 1, which comprises the microorganism Streptomyces purpurascens Y-11472 (DSM 2658) being fermented in a customary manner under aerobic conditions in the presence of a nutrient medium, and the compounds formed being isolated from the culture liquid and from the mycelium, in a customary manner by extraction with organic solvents followed by chromatography or partition.

10. A medicament which contains a compound as claimed in claim 1.

11. The use of a compound as claimed in claim 1, for the preparation of a medicament having cytostatic activity.

12. The microorganism Streptomyces purpurascens Y-11472 (DSM 2658).

**Claims** (for the Contracting State AT)

1. A process for the preparation of a compound of the formula I

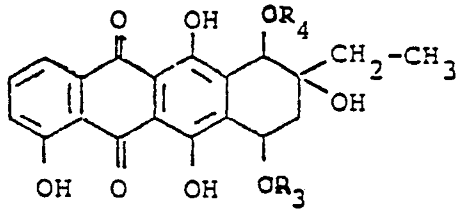

(I)

in which $R_3$ has the meaning Roa-Rod-Rod, Roa-dF-Cin A or Roa-Rod-Acu if $R_4$ represents the sugar combination Roa-Rod-Rod, or $R_3$ has the meaning Roa-Rod-Acu or Roa-dF-Cin A if $R_4$ represents the sugar combination Roa-Rod-Acu, or $R_3$ has the meaning Roa-Rod-Rod or Roa-dF-Rod if $R_4$ represents the sugar combination Roa-dF-Rod,
in which
Roa denotes rhodosamine,
dF denotes deoxyfucose,
Rod denotes rhodinose,
Acu denotes aculose, and
Cin A denotes cinerulose A
and its physiologically acceptable acid addition salts which comprises the microorganism Streptomyces purpurascens Y-11472 (DSM 2658) being fermented in a customary manner under aerobic conditions in the presence of a nutrient medium, and the compounds formed being isolated from the culture liquid and from the mycelium, in a customary manner by extraction with organic solvents followed by chromatography or partition.

2. The process as claimed in claim 1, wherein the microorganism Streptomyces purpurascens is fermented in a nutrient medium containing sources of carbon and nitrogen and inorganic nutrient salts and trace elements, at temperatures of 24-40 °C and a pH of 6.5-8.5, the compounds formed are extracted from the culture liquid using ethyl acetate or chloroform, and are first extracted from the mycelium using aqueous acetone and then extracted with ethyl acetate from the aqueous phase which has been separated off, the combined ethyl acetate extracts are concentrated and the residue is taken up in benzene or toluene, the resulting solution is extracted with an acetate buffer at a pH of 3.5, and the compounds of the formula I are isolated from the resulting solution by chromatography or partition.

3. The process as claimed in claim 1 and 2, wherein the compounds of the formula I are isolated by chromatography on silica gel columns, reversed phase adsorbants, by droplet countercurrent chromatography or partition and subsequent thin-layer or high-pressure liquid chromatography.

4. The process as claimed in claim 1, wherein $R_3$ represents the sugar combination Roa-dF-Cin A and $R_4$ represents the sugar combination Roa-Rod-Rod.

5. The process as claimed in claim 1, wherein $R_3$ and $R_4$ represent the sugar combination Roa-Rod-Rod.

6. The process as claimed in claim 1, wherein $R_3$ represents the sugar combination Roa-Rod-Acu, and $R_4$ represents the sugar combination Roa-Rod-Rod.

7. The process as claimed in claim 1, wherein $R_3$ and $R_4$ represent the sugar combination Roa-Rod-Acu.

8. The process as claimed in claim 1, wherein $R_3$ represents the sugar combination Roa-dF-Cin A and $R_4$ represents the sugar combination Roa-Rod-Acu.

9. The process as claimed in claim 1, wherein $R_3$ represents the sugar combination Roa-Rod-Rod, and $R_4$ represents the sugar combination Roa-dF-Rod.

10. A process as claimed in claim 1, wherein $R_3$ and $R_4$ represent the sugar combination Roa-dF-Rod.

11. The use of a compound as claimed in claim 1 for the preparation of a medicament having cycostatic activity.

**Revendications** (pour les Etats contractants : BE, CH, LI, DE, FR, GB, IT, LU, NL, SE)

1. Composés de formule

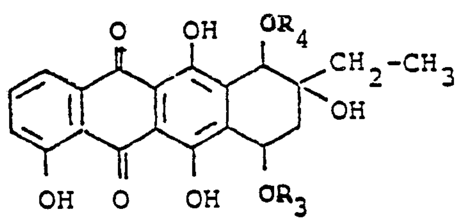

dans laquelle, lorsque $R_4$ représente la combinaison de sucres Roa-Rod-Rod, $R_3$ a la signification de Roa-Rod-Rod, Roa-dF-Cin A ou Roa-Rod-Acu, ou, lorsque $R_4$ représente la combinaison de sucres Roa-Rod-Acu, $R_3$ a la signification de Roa-Rod-Acu ou Roa-dF-Cin A, ou, lorsque $R_4$ représente la combinaison de sucres Roa-dF-Rod, $R_3$ a la signification de Roa-Rod-Rod ou Roa-dF-Rod,
Roa signifiant la rhodosamine,
dF signifiant la désoxyfucose,
Rod signifiant le rhodinose,

Acu signifiant l'aculose et

Cin A signifiant le cinérulose A,

et sels d'addition avec des acides physiologiquement acceptables de ceux-ci.

2. Composé selon la revendication 1, dans lequel $R_3$ représente la combinaison de sucres Roa-dF-Cin A, et $R_4$, la combinaison de sucres Roa-Rod-Rod, et sels d'addition avec des acides physiologiquement acceptables de celui-ci.

3. Composé selon la revendication 1, dans lequel $R_3$ et $R_4$ représentent la combinaison de sucres Roa-Rod-Rod, et sels d'addition avec des acides physiologiquement acceptables de celui-ci.

4. Composé selon la revendication 1, dans lequel $R_3$ représente la combinaison de sucres Roa-Rod-Acu, et $R_4$, la combinaison de sucres Roa-Rod-Rod, et sels d'addition avec des acides physiologiquement acceptables de celui-ci.

5. Composé selon la revendication 1, dans lequel $R_3$ et $R_4$ représentent la combinaison de sucres Roa-Rod-Acu, et sels d'addition avec des acides physiologiquement acceptables de celui-ci.

6. Composé selon la revendication 1, dans lequel $R_3$ représente la combinaison de sucres Roa-dF-Cin A, et $R_4$, la combinaison de sucres Roa-Rod-Acu, et sels d'addition avec des acides physiologiquement acceptables de celui-ci.

7. Composé selon la revendication 1, dans lequel $R_3$ représente la combinaison de sucres Roa-Rod-Rod, et $R_4$, la combinaison de sucres Roa-dF-Rod, et sels d'addition avec des acides physiologiquement acceptables de celui-ci.

8. Composé selon la revendication 1, dans lequel $R_3$ et $R_4$ représentent la combinaison de sucres Roa-dF-Rod, et sels d'addition avec des acides physiologiquement acceptables de celui-ci.

9. Procédé pour la production de composés selon la revendication 1, caractérisé en ce que l'on cultive par fermentation le micro-organisme Streptomyces purpurascens Y-11472 (DSM 2658) de façon usuelle, dans des conditions aérobies, en présence d'un milieu nutritif, et on isole de façon usuelle, à partir du liquide de culture et du mycélium, les composés formés, par extraction avec des solvants organiques et chromatographie subséquente ou partage subséquent.

10. Médicament caractérisé par une teneur en un composé selon la revendication 1.

11. Utilisation d'un composé selon la revendication 1, pour la fabrication d'un médicament à action cytostatique.

12. Le micro-organisme Streptomyces purpurascens Y-11472 (DSM 2658).

**Revendications** (pour l'Etat contractant AT)

1. Procédé pour la production de composés de formule I

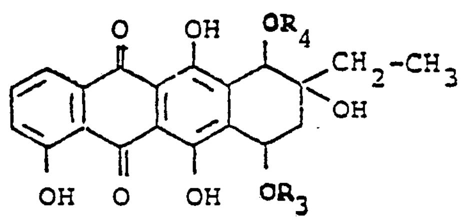

dans laquelle, lorsque $R_4$ représente la combinaison de sucres Roa-Rod-Rod, $R_3$ a la signification de Roa-Rod-Rod, Roa-dF-Cin A ou Roa-Rod-Acu, ou, lorsque $R_4$ représente la combinaison de sucres Roa-Rod-Acu, $R_3$ a la signification de Roa-Rod-Acu ou Roa-dF-Cin A, ou, lorsque $R_4$ représente la combinaison de sucres Roa-dF-Rod, $R_3$ a la signification de Roa-Rod-Rod ou Roa-dF-Rod,

Roa signifiant la rhodosamine,

dF signifiant le désoxyfucose,

Rod signifiant le rhodinose,

Acu signifiant l'aculose et

Cin A signifiant le cinérulose A,

ainsi que de leurs sels d'addition avec des acides physiologiquement acceptables, caractérisé en ce que l'on cultive par fermentation le micro-organisme Streptomyces purpurascens Y-11472 (DSM 2658) de façon usuelle, dans des conditions aérobies, en présence d'un milieu nutritif, et on isole de façon usuelle, à partir du liquide de culture et du mycélium, les composés formés, par extraction avec des solvants organiques et chromatographie subséquente ou partage subséquent.

2. Procédé selon la revendication 1, caractérisé en ce que l'on cultive par fermentation le micro-organisme Streptomyces purpurascens dans un milieu de culture contenant des sources de carbone et d'azote ainsi que des sels minéraux nutritifs et des oligoéléments, à des températures de 24 à 40 °C et à un pH de 6,5 à 8,5, on extrait les composés formés, à partir du liquide de culture, avec de l'acétate d'éthyle ou du chloroforme, et, à partir du mycélium, d'abord avec de l'acétone en solution aqueuse, ensuite on les extrait avec de l'acétate d'éthyle hors de la phase aqueuse séparée, on concentre les extraits d'acétate

d'éthyle réunis, puis on reprend le résidu avec du benzène ou du toluène, on extrait la solution obtenue avec un tampon acétate à un pH de 3, 5, et à partir de la solution obtenue on isole les composés de formule I par chromatographie ou partage.

3. Procédé selon la revendication 1 ou 2, caractérisé en ce que l'on effectue l'isolement chromatographiquement des composés de formule I par chromatographie sur colonne de gel de silice, chromatographie sur adsorbants « en phases inversées », chromatographie en gouttelettes à contre-courant, ou par partage et chromatographie sur couche mince ou chromatographie liquide à haute pression.

4. Procédé selon la revendication 1, dans lequel $R_3$ représente la combinaison de sucres Roa-dF-Cin A, et $R_4$, la combinaison de sucres Roa-Rod-Rod.

5. Procédé selon la revendication 1, dans lequel $R_3$ et $R_4$ représentent la combinaison de sucres Roa-Rod-Rod.

6. Procédé selon la revendication 1, dans lequel $R_3$ représente la combinaison de sucres Roa-Rod-Acu, et $R_4$, la combinaison de sucres Roa-Rod-Rod.

7. Procédé selon la revendication 1, dans lequel $R_3$ et $R_4$ représentent la combinaison de sucres Roa-Rod-Acu.

8. Procédé selon la revendication 1, dans lequel $R_3$ représente la combinaison de sucres Roa-dF-Cin A, et $R_4$, la combinaison de sucres Roa-Rod-Acu.

9. Procédé selon la revendication 1, dans lequel $R_3$ représente la combinaison de sucres Roa-Rod-Rod, et $R_4$, la combinaison de sucres Roa-dF-Rod.

10. Procédé selon la revendication 1, dans lequel $R_3$ et $R_4$ représentent la combinaison de sucres Roa-dF-Rod.

11. Utilisation d'un composé selon la revendication 1, pour la fabrication d'un médicament à action cytostatique.

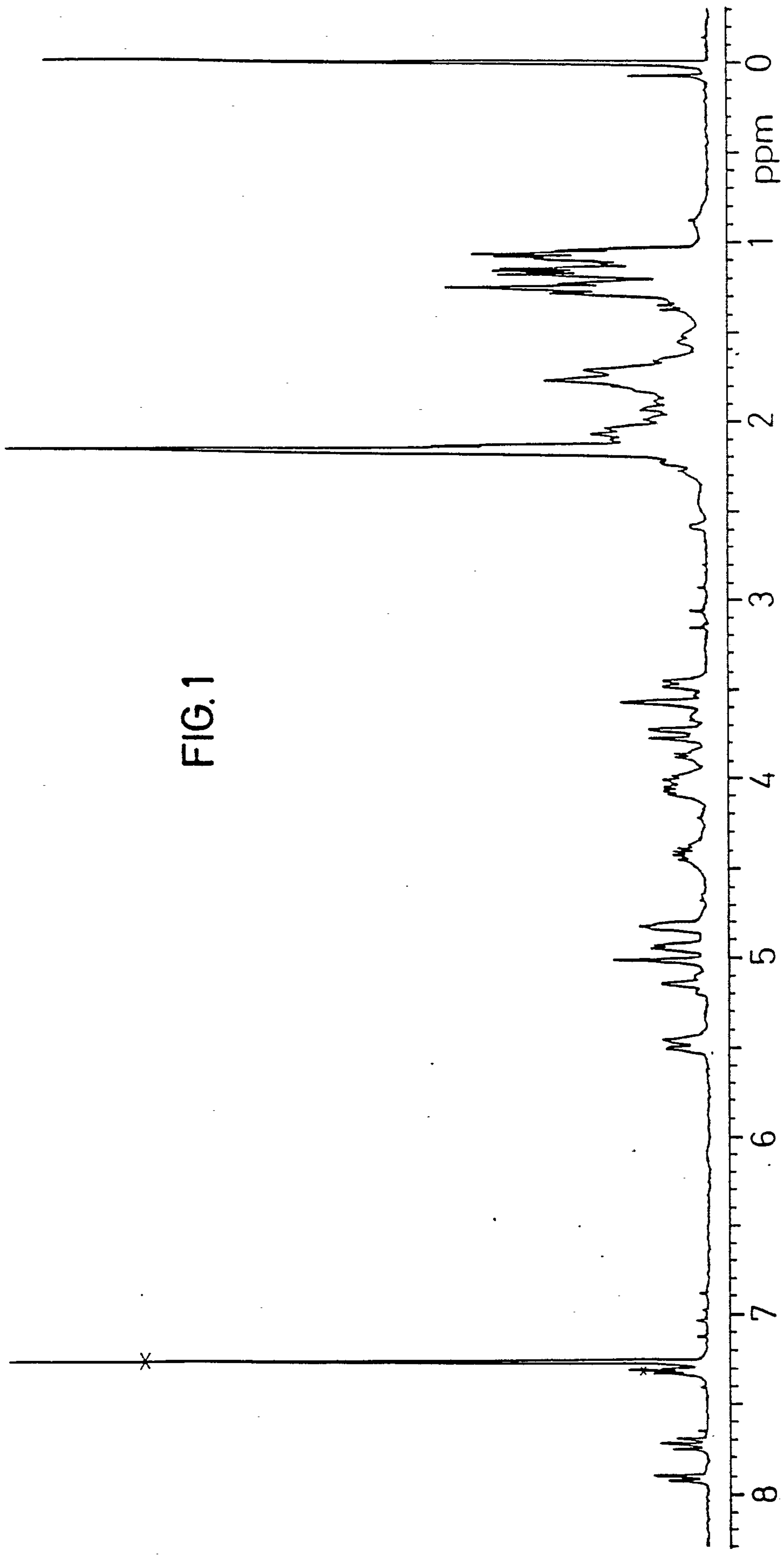


FIG.1

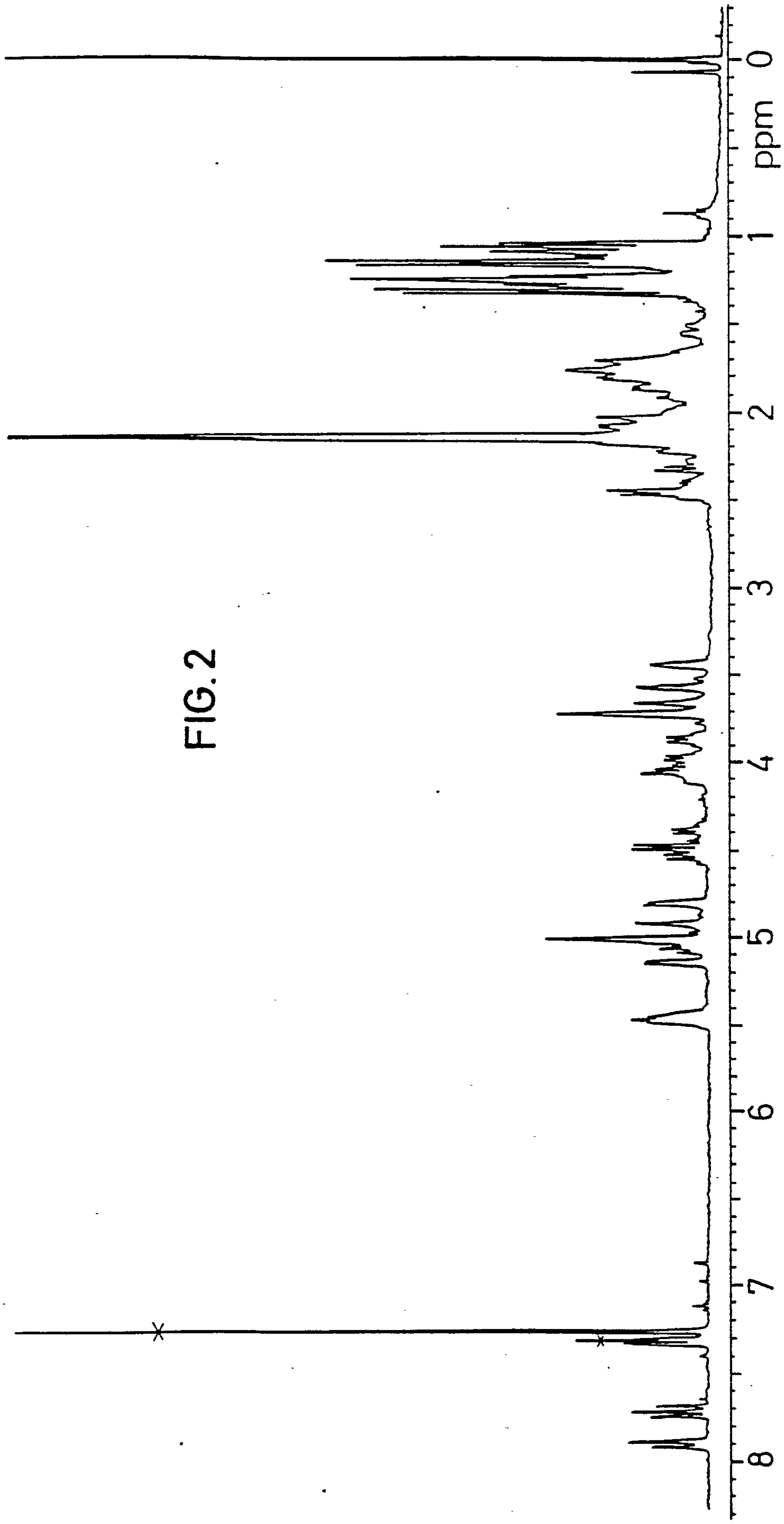


FIG. 2

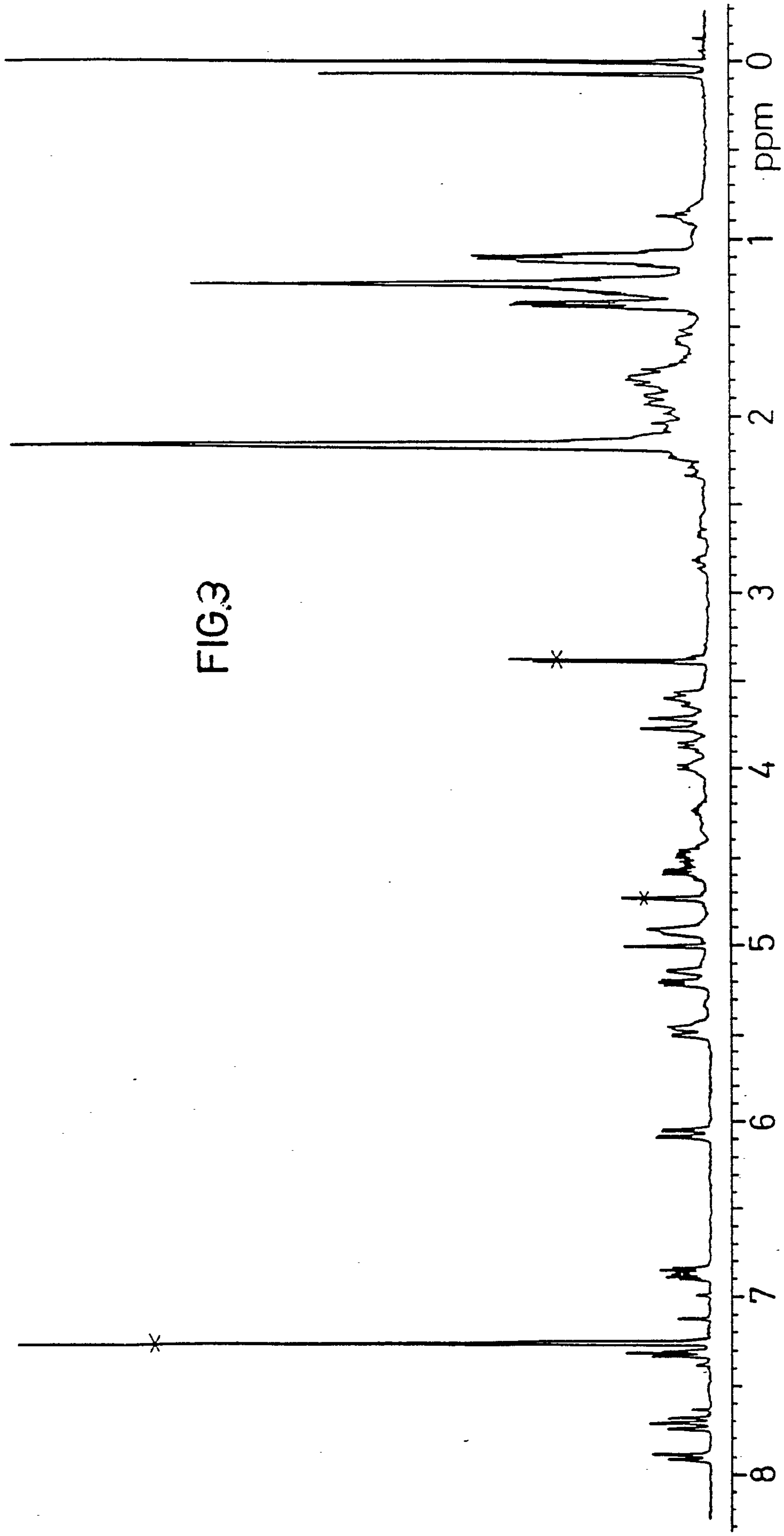


FIG.3

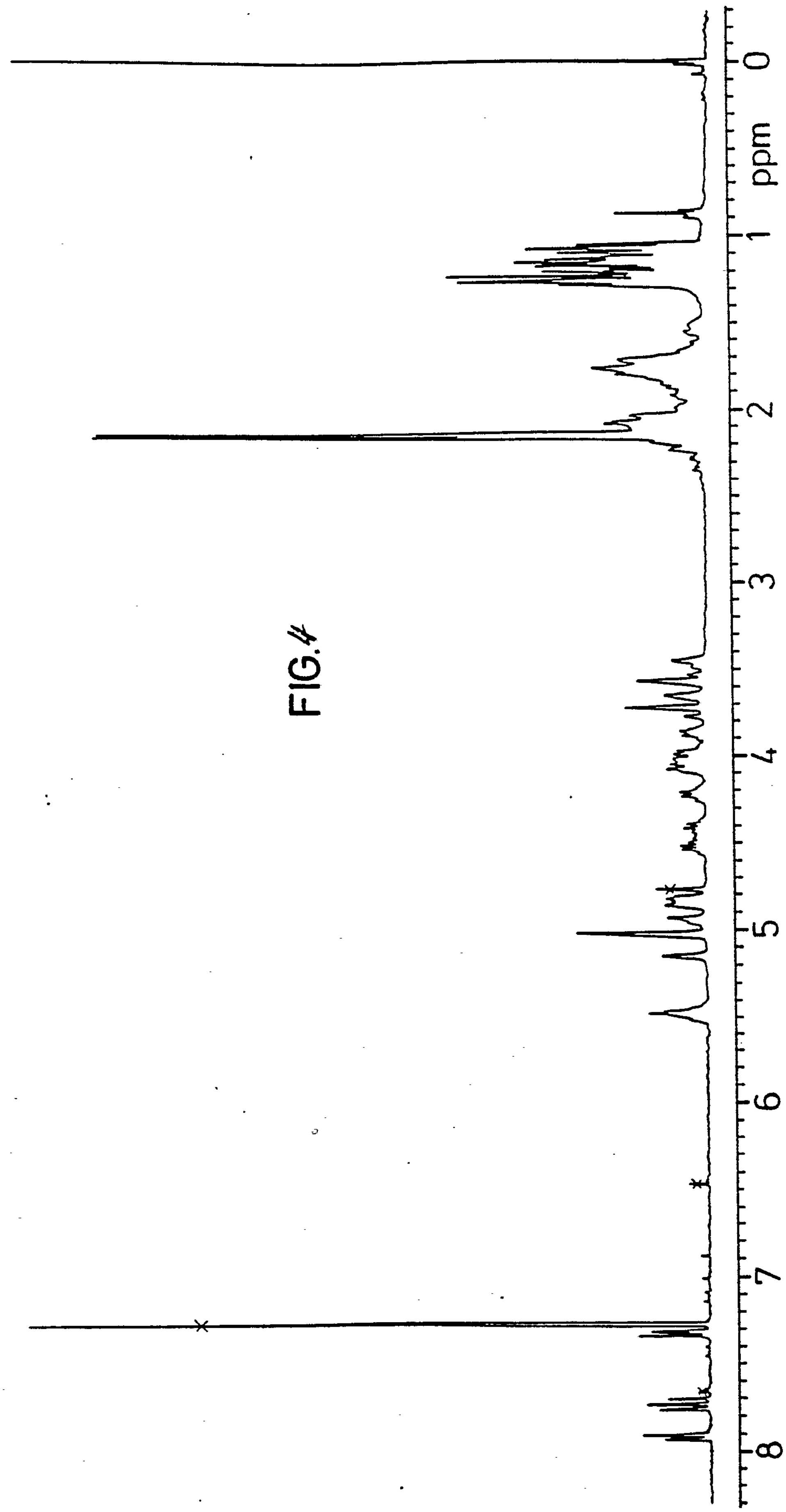


FIG.4

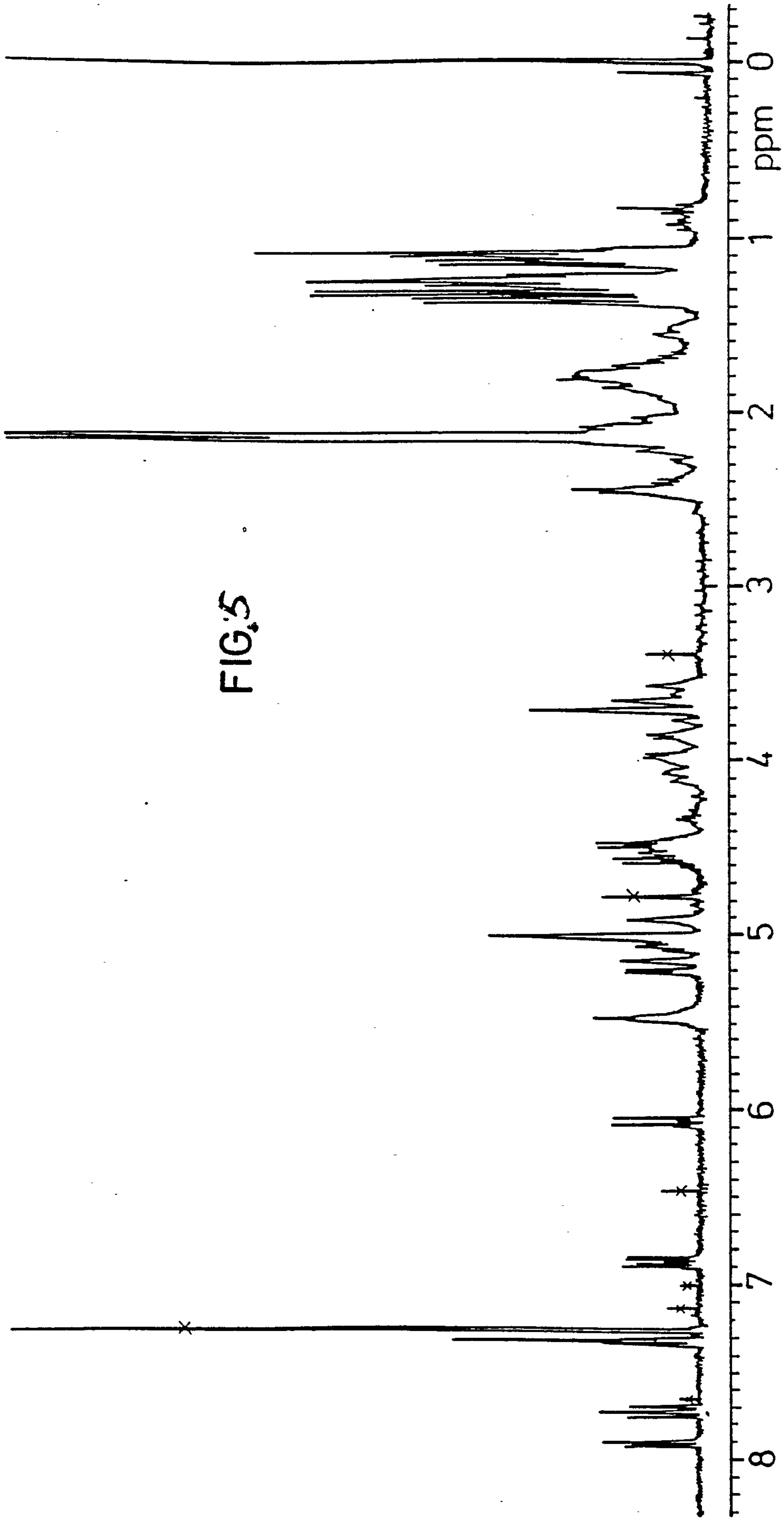


FIG. 5

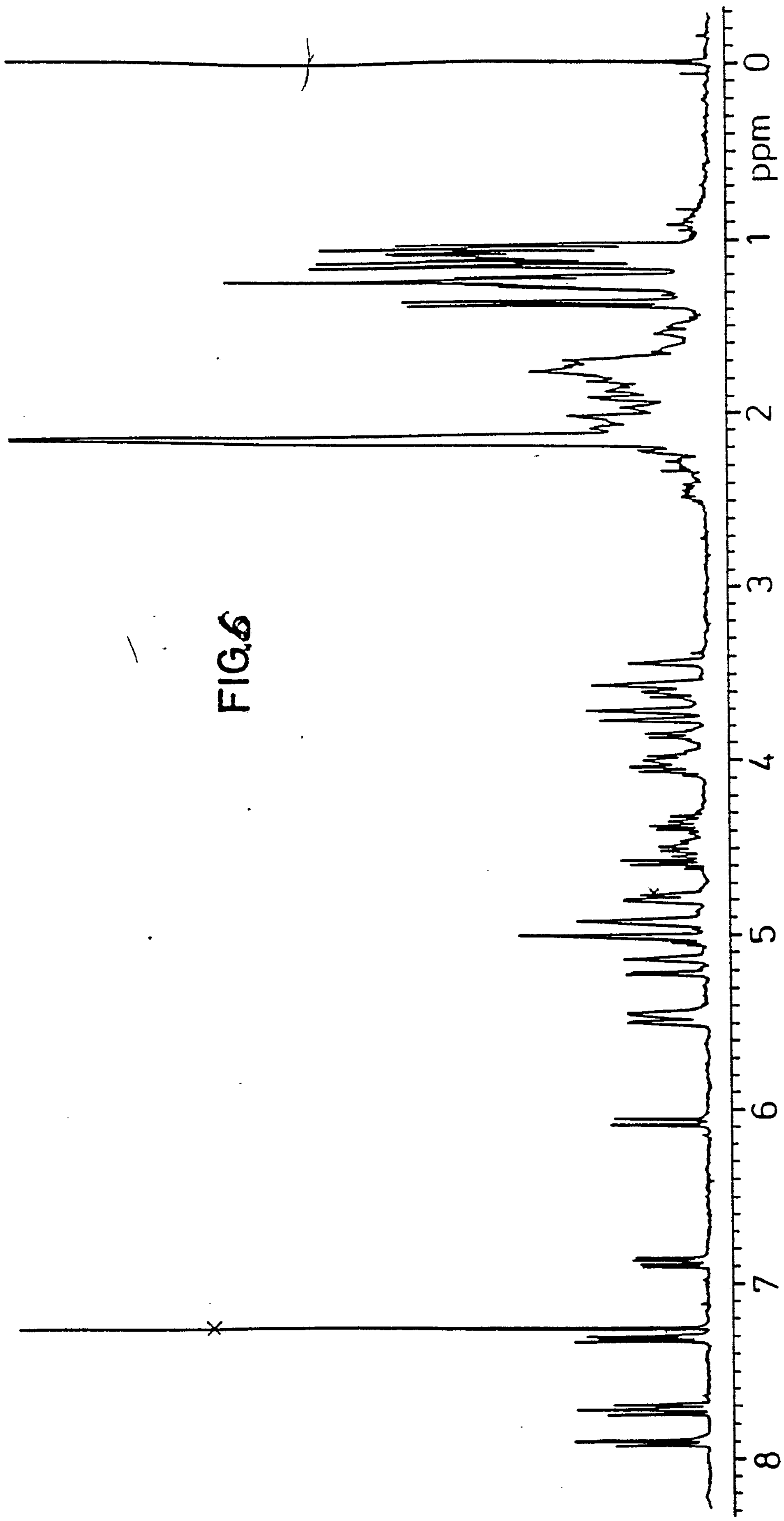


FIG.6